# EUROPEAN PATENT SPECIFICATION

(11) **EP 1 185 639 B1**
(45) Date of publication and mention of the grant of the patent: **05.05.2010**
(21) Application number: 00918373.2
(22) Date of filing: 24.03.2000
(51) Int. Cl.: C12N 15/12, C12N 15/11, C12N 9/00, C12Q 1/68, A61K 48/00

(54) **PROSTATE-SPECIFIC GENE, PCGEM1, AND METHODS OF USING PCGEM1 TO DETECT, TREAT, AND PREVENT PROSTATE CANCER**
PROSTATA-SPEZIFISCHES GEN PCGEM1 UND METHODEN ZU DESSEN VERWENDUNG ZUR ERKENNUNG, BEHANDLUNG UND PRÄVENTION VON PROSTATAKREBS
GENE PROSTATIQUE SPECIFIQUE, FAMILLE DE GENES PCGEM1, ET METHODES D'EMPLOI DE PCGEM1 POUR LA DETECTION, LE TRAITEMENT ET LA PREVENTION DU CANCER DE LA PROSTATE

(30) Priority: 26.03.1999 US 126469 P
(43) Date of publication of application: 13.03.2002
(73) Proprietor: HENRY M. JACKSON FOUNDATION FOR THE ADVANCEMENT OF MILITARY MEDICINE, Rockville, MD 20852 (US)
(72) Inventor: SRIKANTAN, Vasantha, Rockville, MD 20851 (US); ZOU, Zhiqiang, Gaithersburg, MD20878 (US); MOUL, Judd W., Bethesda,MD 20817 (US); SRIVASTAVA,Shiv, Potomac, MD 20854 (US)
(74) Representative: MacLean, Martin Robert
(86) International application number: PCT/US2000/007906
(87) International publication number: WO 2000/058470

(56) References cited:
- WO-A-95/19434
- WO-A-99/00498
- "AC AC003046" EBI DATABASE, XP002143197
- SRIKANTAN V ET AL: "Structure and expression of a novel prostate specific gene: PC-GEM1." PROCEEDINGS OF THE AMERICAN ASSOCIATION FOR CANCER RESEARCH ANNUAL, vol. 40, March 1999 (1999-03), page 37 XP000929230 90th Annual Meeting of the American Association for Cancer Research;Philadelphia, Pennsylvania, USA; April 10-14, 1999, March, 1999 ISSN: 0197-016X
- BUSSEMAKERS M J G ET AL: "A NEW PROSTATE-SPECIFIC MARKER, STRONGLY OVEREXPRESSED IN PROSTATIC TUMORS" UROLOGICAL RESEARCH,DE,SPRINGER VERLAG, BERLIN, vol. 25, no. 1, 1 February 1997 (1997-02-01), page 76 XP002074305 ISSN: 0300-5623
- WANG ZHOU ET AL: "Genes regulated by androgen in the rat ventral prostate." PROCEEDINGS OF THE NATIONAL ACADEMY OF SCIENCES OF THE UNITED STATES, vol. 94, no. 24, 25 November 1997 (1997-11-25), pages 12999-13004, XP002143199 Nov. 25, 1997 ISSN: 0027-8424
- "AC AC013401" EBI DATABASE, XP002143200
- DATABASE EMBL [Online] 28 October 1998 (1998-10-28), Database accession no. AF099810
- DATABASE EMBL [Online] 11 March 1999 (1999-03-11), Database accession no. AC006925

### SEQUENCE LISTING

<110> Srikantan, Vasantha
   Zou, Zhiqiang
   Moul , Judd W.
   Srivastava, Shiv
<120> PROSTATE-SPECIFIC GENE, PCGEM1, AND METHODS OF USING
   PCGEM1 TO DETECT, TREAT, AND PREVENT PROSTATE CANCER
<130> 4995.0053-003-04
<140> >
   <141> >
<150> 60/126,469 <151> 1999-03-26
<160> 22
<170> PatentIn Ver. 2.1
<210> 1
   <211> 1603
   <212> DNA
   <213> Homo sapiens
<400> 1
   aaggcactct ggcacccagt tttggaactg cagttttaaa agtcataaat tgaatgaaaa 60
   tgatagcaaa ggtggaggtt tttaaagagc tatttatagg tccctggaca gcatcttttt 120
   tcaattaggc agcaaccttt ttgccctatg ccgtaacctg tgtctgcaac ttcctctaat 180
   tgggaaatag ttaagcagat tcatagagct gaatgataaa attgtactac gagatgcact 240
   gggactcaac gtgaccttat caagtgagca ggcttggtgc atttgacact tcatgatatc 300
   atccaaagtg gaactaaaaa cagctcctgg aagaggacta tgacatcatc aggttgggag 360
   tctccaggga cagcggaccc tttggaaaag gactagaaag tgtgaaatct attagtcttc 420
   gatatgaaat tctctgtctc tgtaaaagca tttcatattt acaagacaca ggcctactcc 480
   tagggcagca aaaagtggca acaggcaagc agagggaaaa gagatcatga ggcatttcag 540
   agtgcactgt cttttcatat atttctcaat gccgtatgtt tggttttatt ttggccaagc 600
   ataacaatct gctcaagaaa aaaaaatctg gagaaaacaa aggtgccttt gccaatgtta 660
   tgtttctttt tgacaagccc tgagatttct gaggggaatt cacataaatg ggatcaggtc 720
   attcatttac gttgtgtgca aatatgattt aaagatacaa cctttgcaga gagcatgctt 780
   tcctaagggt aggcacgtgg aggactaagg gtaaagcatt cttcaagatc agttaatcaa 840
   gaaaggtgct ctttgcattc tgaaatgccc ttgttgcaaa tattggttat attgattaaa 900
   tttacactta atggaaacaa cctttaactt acagatgaac aaacccacaa aagcaaaaaa 960
   tcaaaagccc tacctatgat ttcatatttt ctgtgtaact ggattaaagg attcctgctt 1020
   gcttttgggc ataaatgata atggaatatt tccaggtatt gtttaaaatg agggcccatc 1080
   tacaaattct tagcaatact ttggataatt ctaaaattca gctggacatt gtctaattgt 1140
   tttttatata catctttgct agaatttcaa attttaagta tgtgaattta gttaattagc 1200
   tgtgctgatc aattcaaaaa cattactttc ctaaatttta gactatgaag gtcataaatt 1260
   caacaaatat atctacacat acaattatag attgtttttc attataatgt cttcatctta 1320
   acagaattgt ctttgtgatt gtttttagaa aactgagagt tttaattcat aattacttga 1380
   tcaaaaaatt gtgggaacaa tccagcatta attgtatgtg attgttttta tgtacataag 1440
   gagtcttaag cttggtgcct tgaagtcttt tgtacttagt cccatgttta aaattactac 1500
   tttatatcta aagcatttat gtttttcaat tcaatttaca tgatgctaat tatggcaatt 1560
   ataacaaata ttaaagattt cgaaatagaa aaaaaaaaaa aaa 1603
<210> 2
   <211> 1579
   <212> DNA
   <213> Homo sapiens
<400> 2
   gcggccgcgt cgacgcaact tcctctaatt gggaaatagt taagcagatt catagagctg 60
   aatgataaaa ttgtacttcg agatgcactg ggactcaacg tgaccttatc aagtgagatg 120
   gagtcttgcc ctgtctccaa ggctggagcc caatggtgtg atcttggctc actgcaacct 180
   ccacctccca ggttcaaacg tttctcctgc ctcagcctcc caagtaactg ggattacagc 240
   aggcttggtg catttgacac ttcatgatat cagccaaagt ggaactaaaa acagctcctg 300
   gaagaggact atgacatcat caggttggga gtctccaggg acagcggacc ctttggaaaa 360
   ggactagaaa gtgtgaaatc tattagtctt cgatatgaaa ttctctgtct ccgtaaaagc 420
   atttcatatt tacaagacac aggcctactc ctagggcagc aaaaagtggc aacaggcaag 480
   cagagggaaa agagatcatg aggcatttca gagtgcactg tcttttcata tatttctcaa 540
   tgccgtatgt ttggttttat tttggccaag cataacaatc tgctcaaaaa aaaaaaatct 600
   ggagaaaaca aaggtgcctt tgccaatgtt atgtttcttt ttgacaagcc ctgagatttc 660
   tgaggggaat tcacataaat gggatcaggt cattcattta cgttgtgtgc aaatatgatt 720
   taaagataca acctttgcag agagcatgct ttcctaaggg taggcacgtg gaggactaag 780
   ggtaaagcat tcttcaagat cagttaatca agaaaggtgc tctttgcatt ctgaaatgcc 840
   cttgttgcaa atattggtta tattgattaa atttacactt aatggaaaca acctttaact 900
   tacagatgaa caaaccccac aaaagcaaaa aatcaaaagc cctacctatg atttcatatt 960
   ttctgtgtaa ctggattaaa ggattcctgc ttgcttttgg gcataaatga taatggaata 1020
   tttccaggta ttgtttaaaa tgagggccca tctacaaatt cttagcaata ctttggataa 1080
   ttctaaaatt cagctggaca ttgtctaatt gttttttata tacatctttg ctagaatttc 1140
   aaattttaag tatgtgaatt tagttaatta gctgtgctga tcaattcaaa aacattactt 1200
   tcctaaattt tagactatga aggtcataaa ttcaacaaat atatctacac atacaattat 1260
   agattgtttt tcattataat gtcttcatct taacagaatt gtctttgtga ttgtttttag 1320
   aaaactgaga gttttaattc ataattactt gatcaaaaaa ttgtgggaac aatccagcat 1380
   taattgtatg tgattgtttt tatgtacata aggagtctta agcttggtgc cttgaagtct 1440
   tttgtactta gtcccatgtt taaaattact actttatatc taaagcattt atgtttttca 1500
   attcaattta catgatgcta attatggcaa ttataacaaa tattaaagat ttcgaaatag 1560
   aaaaaaaaaa aaaaatcta                                                1579
<210> 3
   <211> 1819
   <212> DNA
   <213> Homo sapiens
<400> 3
   tccctcttgc gttctgcaat ttctgaaaaa aagatgttta ttgcaaagtg atatgagcac 60
   tggaaaggta ctaattccaa tttgattcta attggatgag tgacatgggt aagcgattct 120
   aagcatttgt gtttttttta gtagtatgga atttaattag ttctcagtat gttagtgaag 180
   atgaatgaaa acatgcatat gtttccatgt attataaata ttttaaaatg caaaaaatta 240
   ttctaatgaa tatataaata taaagcataa caataataat acaataccac ccataaagtc 300
   atcatctaat ttaaaaacta aaacattaac acttgaatct cccccattgc aacatctttc 360
   ccgacttgtg tgtttttttc ttttgctttt aaaatttttg ttttatcata tgtctgcata 420
   agattatata gctttccttg ttttaagctt tttaaataat atattgtagt tatattattt 480
   gtgctttgct ttttttactt aacattatgg ttctaaaatt cagtaatgtg ttgggcatgt 540
   ataatttgtt tatttttaat ctctttgaca ttcgactata taaatttcag tttgtttatt 600
   gactcctttg tctatagata ctctgctatt tctgtttttg ctgttacaaa aataatgctg 660
   ttttaaattt cattttgtat acttttttga ggcatgtgta tgagttattc taaggtaaaa 720
   aaataagaaa aaattgctgg gttataagat tgtcacatgc tcgaatttac aagataatgc 780
   caaatcattt ttcaaagtaa ttatacctat ttatactacc ggtatgagta tattggtgcc 840
   cacatagttg cttgttctgc caaagtttgg tatgatcgaa caataatttt tgcccatcaa 900
   atggcataaa ataaaatctc agtgtgcttt taatttgcat tttctatgtt taagaattgt 960
   ttctttttta accatttata atttactttt gctgaaatgc ttgcttatta tttttgctcc 1020
   ccattttttc ctattggatt gcttttctca ttaatttata agaattttat atggtttaga 1080
   tactaattat tatattactg aaaatacctt tatcagtttg ttgtgtactt tctactttat 1140
   gtcttgtgat ggataaaagt tttaaattgt attgtgttga agttaacatt tttaaatttt 1200
   ataatcagca tctttaataa tctctttmta aaattttcct ttacatagat gtcataaaga 1260
   tacatctcta taatttctta tttttttggc atatgttcat taagtcattt tatcattttt 1320
   tagtaataaa ttgcagttat ttatgaaaca aataattttt aaaattatat atgctttctt 1380
   taaaaattga tcttagcatg cttcactatg aagcttgagg cttcactgca cgttgtactg 1440
   aaattatgta taaaacagtg gttctgaaaa tctctgagtt catgacacct ttagtgtctc 1500
   aggttttttt gcttttgttc ttgttttttc tcacaaagca cctaagttaa ataaaaacaa 1560
   agcacaaagc tatcagcttc atgtattaag tagtaagctc ccatgttaac agttgtaact 1620
   tgcctggtgc ccaatagatg tcactctgtt ttcctagaaa ctttaaaata tccctcagtg 1680
   ctcctgttaa ttcatggtag tgccccaagg cactctggca cccagttttg gaactgcagt 1740
   tttaaaagtc ataaattgaa tgaaaatgat agcaaaggtg gaggttttta aagagctatt 1800
   tataggtccc tggacagca 1819
<210> 4
   <211> 1025
   <212> DNA
   <213> Homo sapiens
<400> 4
   ttttttcaat taggcagcaa cctttttgcc ctatgccgta acctgtgtct gcaacttcct 60
   ctaattggga aatagttaag cagattcata gagctgaatg ataaaattgt actacgagat 120
   gcactgggac tcaacgtgac cttatcaagt gagcaggctt ggtgcatttg acacttcatg 180
   atatcatcca aagtggaact aaaaacagct cctggaagag gactatgaca tcatcaggtt 240
   gggagtctcc agggacagcg gaccctttgg aaaaggacta gaaagtgtga aatctattag 300
   tcttcgatat gaaattctct gtctctgtaa aagcatttca tatttacaag acacaggcct 360
   actcctaggg cagcaaaaag tggcaacagg caagcagagg gaaaagagat catgaggcat 420
   ttcagagtgc actgtctttt catatatttc tcaatgccgt atgtttggtt ttattttggc 480
   caagcataac aatctgctca agaaaaaaaa atctggagaa aacaaaggtg cctttgccaa 540
   tgttatgttt ctttttgaca agccctgaga tttctgaggg gaattcacat aaatgggatc 600
   aggtcattca tttacgttgt gtgcaaatat gatttaaaga tacaaccttt gcagagagca 660
   tgctttccta agggtaggca cgtggaggac taagggtaaa gcattcttca agatcagtta 720
   atcaagaaag gtgctctttg cattctgaaa tgcccttgtt gcaaatattg gttatattga 780
   ttaaatttac acttaatgga aacaaccttt aacttacaga tgaacaaacc cacaaaagca 840
   aaaaatcaaa agccctacct atgatttcat attttctgtg taactggatt aaaggattcc 900
   tgcttgcttt tgggcataaa tgataatgga atatttccag gtattgttta aaatgagggc 960
   ccatctacaa attcttagca atactttgga taattctaaa attcagctgg acattgtcta 1020
   attgt                                                             1025
<210> 5
   <211> 21
   <212> DNA
   <213> Artificial Sequence
<220>
   <223> Description of Artificial Sequence:Probe/Primer
<400> 5
   tgcctcagcc tcccaagtaa c 21
<210> 6
   <211> 21
   <212> DNA
   <213> Artificial Sequence
<220>
   <223> Description of Artificial Sequence:Probe/Primer
<400> 6
   ggccaaaata aaaccaaaca t 21
<210> 7
   <211> 19
   <212> DNA
   <213> Artificial Sequence
<220>
   <223> Description of Artificial Sequence:Probe/Primer
<400> 7
   tggcaacagg caagcagag 19
<210> 8
   <211> 11801
   <212> DNA
   <213> Homo sapiens
<220>
   <221> unsure
   <222> (7470)
   <223> Y may represent any of the four nucleotide bases
<400> 8
   tccctcttgc gttctgcaat ttctgaaaaa aagatgttta ttgcaaagtg atatgagcac 60
   tggaaaggta ctaattccaa tttgattcta attggatgag tgacatgggt aagcgattct 120
   aagcatttgt gtttttttta gtagtatgga atttaattag ttctcagtat gttagtgaag 180
   atgaatgaaa acatgcatat gtttccatgt attataaata ttttaaaatg caaaaaatta 240
   ttctaatgaa tatataaata taaagcataa caataataat acaataccac ccataaagtc 300
   atcatctaat ttaaaaacta aaacattaac acttgaatct cccccattgc aacatctttc 360
   ccgacttgtg tgtttttttc ttttgctttt aaaatttttg ttttatcata tgtctgcata 420
   agattatata gctttccttg ttttaagctt tttaaataat atattgtagt tatattattt 480
   gtgctttgct ttttttactt aacattatgg ttctaaaatt cagtaatgtg ttgggcatgt 540
   ataatttgtt tatttttaat ctctttgaca ttcgactata taaatttcag tttgtttatt 600
   gactcctttg tctatagata ctctgctatt tctgtttttg ctgttacaaa aataatgctg 660
   ttttaaattt cattttgtat acttttttga ggcatgtgta tgagttattc taaggtaaaa 720
   aaataagaaa aaattgctgg gttataagat tgtcacatgc tcgaatttac aagataatgc 780
   caaatcattt ttcaaagtaa ttatacctat ttatactacc ggtatgagta tattggtgcc 840
   cacatagttg cttgttctgc caaagtttgg tatgatcgaa caataatttt tgcccatcaa 900
   atggcataaa ataaaatctc agtgtgcttt taatttgcat tttctatgtt taagaattgt 960
   ttctttttta accatttata atttactttt gctgaaatgc ttgcttatta tttttgctcc 1020
   ccattttttc ctattggatt gcttttctca ttaatttata agaattttat atggtttaga 1080
   tactaattat tatattactg aaaatacctt tatcagtttg ttgtgtactt tctactttat 1140
   gtcttgtgat ggataaaagt tttaaattgt attgtgttga agttaacatt tttaaatttt 1200
   ataatcagca tctttaataa tctctttata aaattttcct ttacatagat gtcataaaga 1260
   tacatctcta taatttctta tttttttggc atatgttcat taagtcattt tatcattttt 1320
   tagtaataaa ttgcagttat ttatgaaaca aataattttt aaaattatat atgctttctt 1380
   taaaaattga tcttagcatg cttcactatg aagcttgagg cttcactgca cgttgtactg 1440
   aaattatgta taaaacagtg gttctgaaaa tctctgagtt catgacacct ttagtgtctc 1500
   aggttttttt gcttttgttc ttgttttttc tcacaaagca cctaagttaa ataaaaacaa 1560
   agcacaaagc tatcagcttc atgtattaag tagtaagctc ccatgttaac agttgtaact 1620
   tgcctggtgc ccaatagatg tcactctgtt ttcctagaaa ctttaaaata tccctcagtg 1680
   ctcctgttaa ttcatggtag tgccccaagg cactctggca cccagttttg gaactgcagt 1740
   tttaaaagtc ataaattgaa tgaaaatgat agcaaaggtg gaggttttta aagagctatt 1800
   tataggtccc tggacagcat cttttttcaa ttaggcagca acctttttgc ctatgccgta 1860
   actgtgtctg cacttcctct aattggggtg agtaagagat tttgttatgt atataatagc 1920
   taagaatata gtaataatgg cttaaatcat ggttattttt aaactactaa catttagaag 1980
   acaaaataaa aatgctttga aaagtataga ggttttagtg taattagcag ggaataatga 2040
   aatgatttga tagggctact cagttttgta taactttggt gctttaagtc tgaatgcaga 2100
   gcatggatgt tgtgatccag cctttatatg ttttccctga agaagattta atttatttgg 2160
   ccttttgaga aacacatttg gcattgtaat atgttttgct tccaggttct atctccaagg 2220
   ataatttgac aaaatcacac ataaatttat tttcagggca cacagtttcc cttttaggga 2280
   actcacagag gtagagagta atacaataat cacatttgaa tattcagtaa gtgaggtcct 2340
   catagatctt atgtgtatgt caccatgtat ataattttgt taatcactag atgtatgaga 2400
   caagaaattt gaggaatctt aactagagat taaaatcagg gatttaaatc aaagaaacat 2460
   ttaaatgcct cctttattat ttaaatacct gcatgggaga atcattgaaa aaaaaataaa 2520
   aagcatacaa cttgggaata ttataaacca agaagaattt gttattctgg ttgatttttt 2580
   tttcaggctc cgcacaggca acttaccttt atctctttgt gatttttatt tcttgttaaa 2640
   atatacagaa atagttaagc agattcatag agctgaatat aaaatttact acgagatgca 2700
   ctgggactca acgtgacctt atcaagtgac ttatcagtga ggtgagcatt cttaattcag 2760
   ataatggaac ttattatcat aatcttttgc ttatgctatt gttgagctta actacttatt 2820
   catatttgca tatgcatatt gagataatat catttcatta atttcagtac tgaacactaa 2880
   tctcctaaga gtaattgtga aagtttcaga ttgcactatt tttaactata tatctgtatg 2940
   ttatcttcat atatgcttga ataacttata agcaattgaa actttcaatt acagtatact 3000
   attgaagcaa atcaactaat atatacacat atccattagc aatagtagat aatttttgta 3060
   aatgtccagc acagttcttc atatgtagag gatgttcaaa ttggctaagt tccttttctc 3120
   tcttaattat tagtattttt cctactgctc tttgtataat tattccttcc tctttagctc 3180
   caatccttac aatctattct taacatagca actgggaaga aagtttttaa acataaacca 3240
   gatgatgtca ctccacccca caaaacttcc actattctct gtcacacata gaaagaaaga 3300
   aaaaaaatat tgaaaaccta caaagacttg ctatgatctg gtccaggctc tccctaaaat 3360
   ttcatgtaat ttccagccac taggcctttc tggctctcct tcaatctcat tagccttttc 3420
   actactacaa gttagactgg gttttggccg aggtatttct ttttttcata ttttgccttt 3480
   gcctagattg ctcttccaat agatattcac aattgcatca tcatttctat atacgtgcta 3540
   aaaggtttcc ttgtccaaaa tagcttcagt gaccacctga tctagaatag tctcgatcaa 3600
   aagtttcttt tccttttcct caccacttga tatttatatc aaacatttat ttgtgtaatt 3660
   tatgtgtttg tttgttttct gtactagcat tatgatgacc atactatttg atgcccccca 3720
   aaaaatactt tcgagaatga cagggcaaag ctaaaataat taaattatat aattttgaca 3780
   taggcactat tgacaaaaag caattgatgt tatgatagtg ttagatctat gaaatagtac 3840
   tatttaaaag taattctctg aaatacaatt ttctaaaact aaaagcagca tatgtacatg 3900
   aaacaccaaa aaacttcctt atatttatca ctggaagatt taaaatagta taagtagtaa 3960
   cttatttaat atatttttga ttatttaatt aattttatag tatccaactc taatataatg 4020
   ccagtggtat ttgttcaaaa tattttaatg ttgtctattt atttttaatt tgcctaaaaa 4080
   ttatcttaaa tgaaaatttt tggttaataa atttgaaaat actgaaaccc tcatctccag 4140
   tctctgtgga tcctaaagtt tttagttgag aaaataattt ttctctagag aatgaagtag 4200
   cttgtaagct tggagaaatt tctgctaaat aaatgatatt atcaactctt attttcttca 4260
   atacgaaata tataaatatt tcagctcata tatttttgca ggtgctatgc ttttgcttcc 4320
   aatcataatt tctgacaaat attttggaag tcaaaacttg tcttctattt tgttatttaa 4380
   aattatatag actacttttg taaaccttta tactatcaaa tcataggcaa tttcagtttg 4440
   atttcattct ggtgcagaat ataagtttat ccaagtaaaa caggagtcac ttcaaaagat 4500
   tcctcccact gactgagata ttccaaagcc aactttgcaa aatttcagaa ttaaatatta 4560
   tacttctttg taccttcatt ttatttgttc aatttttctt tgtgtttgta gaaaatttta 4620
   atatttttct gttttcaagt tttgatttta atttactact ttataatttt taaaggtaag 4680
   ttttgtgagg ctatattcat tatgtgtttt gaataaagac atacaattaa ttttgagaac 4740
   tgcaataaaa attataagac tattaaaaat gcagtaagtg tactacactt aggctgctaa 4800
   aaatgcagta ccagtagact acatttaggc tgcttaaagt tagttcttct aagtaccata 4860
   tactttaaaa ttttagctaa tgatggagaa caaagacaga aagactgtgt taccatattc 4920
   tagttggcca ttttgttttg ttttgagaga cgtcacatca gccttatcat aaaaattatt 4980
   tggttttacc attttgactg tgagcaaaat atacagcata atatacaaaa taaaatatat 5040
   gtacatcttc acaacttctt gtttaggatg caattatata tatatatata tatatattta 5100
   ttattatact ttaagttcta gggtacatgg caccacgtgc aggttgttac atatgtatac 5160
   atgtgccatg ttggtgtgct gcacccatta actcgtcatt tacattaggt gtatctccta 5220
   atgctatccc tcccctctct ccccacccca caacaagccc cggtgtgtga tgttcccctt 5280
   cctgtgtcca tgtgttctca ttgttcaatt cccacctatg agtgagaaca cgcagtgttt 5340
   gcttttttgt ccttgcaata gtttgctgag aatgatggtt tccagcttca tccatgtccc 5400
   tacaaaggac atgaactcat cattttttat ggctgcatag tattccatgg tgtatatgtg 5460
   ccaccatttt cttaatccga gtctgtccat tgttgttgga catttgggtt gcaattttga 5520
   gtttcatgtg tagcatgtat agcacaacca attaagattt ctttctttct ctcttttttt 5580
   tttttttttg ttgaaatgga gtcttgcctg tctccaaggc tggagcccaa tggtgtgatc 5640
   ttggcttact gcaacctcca cctcccgggt tcaagcgatt ctcctgcctc agccatccga 5700
   gtagctggga ctataggcgt gcaccaccat gcccagctaa tttttgtatt tttagtacag 5760
   acggggtttc accacggtgg ccaggatggt ctcaatttct tgacctcatg attcacccgc 5820
   cttggcctcc caaagtgctg ggattacagg tgtgaaccac caagcccggc ctgtcacaag 5880
   tttttagtgt tctattttaa tacagaaatt agataaatcc aaagagaaag acatttcata 5940
   tgtgcgtaga gttgtcggaa gaaatgagag tcttataaat aactttaaaa attgtgaaga 6000
   aataaaggca aaatagtcct atgcagtttg atttaaatat attcttaata agagctactt 6060
   ttgtgaaaac cagaatattg aaacatgtag atatggatct tcattagtga ctgacataat 6120
   atattgttat tgttactatt ttattgtatc agccaactaa tattgagtgc tttgtgtatc 6180
   ctaagcacta tgctaaacac tgtaccagta ttacctgata taatcatatt aatatttatt 6240
   atttcacttt tcatatgaaa aaattgaagc acagattaag acactccgaa atcatacctc 6300
   tattgattat cagcaccagg atttgaattg aggcactctg atccagagaa gcttttgttt 6360
   ccatgaaggc ttatgttggg gaaaaataat caaattgcct gtacctcagt tgtataaata 6420
   agaggttggg ttggtagatg attctggctg attcagcaga aaagaaattt attcaaagga 6480
   tatcacacag ttttcataac agttaagaat acagaggaaa cagggcacca gggctaagta 6540
   cagaccaaag tccaaaacca ctgccaaagt tgcagcaagg agaacagcac aaatttgctt 6600
   gctgtcaccc gccactagat gcttttgttt ggagccttga acttgactta cactgccact 6660
   gacatcagca ccagtgctct ctgtgtacta ggaggtggag ttggtgacgt tgctgaactc 6720
   aaagcagatg tttctgctgt gaaatagata cctaatacag aacctgcttc ctcattcatt 6780
   ccctccccaa atcatatgct tgtagtgtgg ctagagtttc tgtttctcct tggtccaggc 6840
   agaatttatg aagcttgcta tttatcgcct taaagattag aagaatattc ataaggtatt 6900
   agattgccat aaggttgaac aaatcaacat tcaacttcaa ggattcaaca ttgttttgtt 6960
   ttcttttggg atacctctgc agcagttcaa atcttatttc tgcccttgga caaccaggtt 7020
   tataaatatt gcagattctc cactgactgc tttgatccta tcttctatat ttatgtatac 7080
   taattagcat ataataaaag attatgttac agaatctcaa aattagtaat tatgaattga 7140
   gatggtgtta tacagtacac taacatccaa gagacttgtt tattccaagg aaaatattta 7200
   gagatattaa atgatatttc tcatccttta gacatataca ttttttagct tacagcctgc 7260
   tttaggcaag caacagactc tcaggatctg ctcctaccag ggtctgaaca tttcctccca 7320
   gttttaaaga aacaaattca aataacattg taacctccag aggaaagttc aagctctttt 7380
   atagtattgt ttaaacagta cagctgagga aactaaagac agagaagtta aatgccttgg 7440
   cacttagtct agatttacaa taaactccty tctacttagg acccactaac aggggctgca 7500
   tttacaccaa aaccatgaag gtggcccaag tcatcactga gaagtagtac aagcaccgag 7560
   ggaatgactt caacaggaac aagaaagcgt ggaaggagat cctagcagga agctccacaa 7620
   gaagatagca tgttacgtct tgcattggat gaagcaggtt cagagagacc tagtgacagc 7680
   tatctccgtc aaggtgcaga aggagagatc attgaatgta gcattttcat gcaaaaaaaa 7740
   aaatgttgaa gtctttggac ttcgggagtc tgtccaaact gcaggtcact cagcctacag 7800
   ttgggatgaa tttcaaaaca ccagttggag ccggttgaat ctttctgcta tgctgtaata 7860
   ttttcagtaa acccagcgca acaacaacaa caaaacacaa aaggaggaga agcagccaag 7920
   tctcttggtt tacagagtag ctcctaatac cccttgctgt ctgtctcaag tgcccaatgg 7980
   gaagatagtc aaaacaatat tcacacctgt gattcatctc tctacatgca gtgtgtgtga 8040
   atctttatat actgcatatt aaggatctgt ctttacagat aaaaactaaa gcattgaagg 8100
   aactccttgt tttgacttat caaagtcctt aagaaaatac tagaaaatta tagccattgt 8160
   ttcaaatttt agctttatat tatcacttga aatgtgatga aatgtggctg atagataata 8220
   attcactgat aacctacaga caattcccat cttaaaatgg accattggat tgaagaatta 8280
   aataaaattg agggttttcc ttacatgttt tgtctaaaga gcgaagtaga aacaactgtt 8340
   catagatctt cattgaggat tcgcatgtga agtaagtact cctaacataa acaagtggac 8400
   ttatcaacca agttccataa atcatgaaca aaaatatttg tccccagaga gactattttt 8460
   ccaccacatc tcttgtaata aacacagagc ccagttcagt taaaatagtt taagggtgga 8520
   cggttcaggg cctgctgagt ggcactcagt aagaaaaccc agcagaacat ttacttctct 8580
   ctttattcca gagcatcaat ggccaaggct ggaagatccc agaacactga acagacattt 8640
   ggtctcttat ggcctgccaa ttttcacagt gggttccaac gctttgggtc aaaccaaaat 8700
   agacctgtta gaaaaatgtc ggttggaata cgctaacaat aagacagaat aaatgtgatt 8760
   atttcacctc atttttatag gacttgagta attttattat aacattcttg agggctggaa 8820
   aatctgaatg ttaggacacc aaatatctcc agaaaacaag ttttatattt ctaatcctgc 8880
   ataataaacc tggggccact gcaggcctca ttaataaaaa cctaatggta taacaataat 8940
   gaggaggaaa tgccaatgcc gcacaaatct gttgagacta aaatatttct caccccagca 9000
   ggcttggtgc atttgacact tcatgatatc agccaaagtg gaactaaaaa cagctcctgg 9060
   aagaggacta tgacatcatc aggttgggag tctccaggga cagcggaccc tttggaaaag 9120
   gactagaaag tgtgaaatct attagtcttc gatatgaaat tctctgtctc tgtcaaaagc 9180
   atttcatatt tacaagacac aggcctactc ctagggcagc aaaaagtggc aacaggcaag 9240
   cagagggaaa agagatcatg aggcatttca gagtgcactg tcttttcata tatttctcaa 9300
   tgccgtatgt ttggttttat tttggccaag cataacaatc tgctcaagaa aaaaaaatct 9360
   ggagaaaaca aaggtgcctt tgccaatgtt atgtttcttt ttgacaagcc ctgagatttc 9420
   tgaggggaat tcacataaat gggatcaggt cattcattta cgttgtgtgc aaatatgatt 9480
   taaagataca acctttgcag agagcatgct ttcctaaggg taggcacgtg gaggactaag 9540
   ggtaaagcat tcttcaagaa tcagttaatc aaagaaaggt gctctttgca ttctgaaatg 9600
   cccttgttgc aaatattggt tatattgatt aaatttacac ttaatggaaa caacctttaa 9660
   cttacagatg aacaaaccca caaaagcaaa aaatcaaaag ccctacctat gatttcatat 9720
   tttctgtgta actggattaa aggattcctg cttgcttttg ggcataaatg ataatggaat 9780
   atttccaggt attgtttaaa atgagggccc atctacaaat tcttagcaat actttggata 9840
   attctaaaat tcagctggac attgtctaat tgttttttat atacatcttt gctagaattt 9900
   caaattttaa gtatgtgaat ttagttaatt agctgtgctg atcaattcaa aaacattact 9960
   ttcctaaatt ttagactatg aaggtcataa attcaacaaa tatatctaca catacaatta 10020
   tagattgttt ttcattataa tgtcttcatc ttaacagaat tgtctttgtg attgttttta 10080
   gaaaactgag agttttaatt cataattacg ttgatcaaaa aattgtggga acaatccagc 10140
   attaattgta tgtgattgtt tttatgtaca taaggagtct taagcttggt gccttgaagt 10200
   cttttgtact tagtcccatg tttaaaatta ctactttata tctaaagcat ttatgttttt 10260
   caattcaatt tacatgatgc taattatggc aattataaca aatattaaag atttcgaaat 10320
   agaatatgtg aattgttcac catacataga aatgaaaagt tcatttcgta aagcaagatg 10380
   ctgggtgaaa gagtgctttt gattgaaaga tcactagatt agtagagggc aagactttta 10440
   gtccctaatc tacccttaat agccatgtgg tcacgtgtaa gtcagtgaac ccatctcatt 10500
   ctcctcatac ttttttcatc tctaaaatga gggtataatt taagctcgtt catttttttt 10560
   tttttttgag atagagtttt gctcttgtca cccaggttgg agtgcaatgg cacgatctca 10620
   gctcactgca accctctgct tcctcggttc aagtgattct ccctgcttca gcctcccaag 10680
   tgagcccggg attacaggtg cccgccacca catctgggcc tagatttttt gtattttcac 10740
   catgttggcc aggctggtct cgaaccccta cctcaggtga tccctcgcct cggcctctca 10800
   aagtgctggg attacaggtg tgagccacca cgcccagccc aatatcagtt tttctttttt 10860
   aacacaaggc taacacaatc aaaatactag ctaggggaga aaaaaaaaat aaggcactgt 10920
   ttatgtgtaa caggctcttg ttgcaatcca ctggggcaga ccaaataaac agtaagaatc 10980
   aaatcctttt catataatcc tttctttgca gaatacataa aatccccaca aatggcttat 11040
   cttccttttt atgatatgtt ggagaattgt agctaagtga cagatatttt gcttgggtgt 11100
   atagaccaca aaggactgtg tcttgatgat ggtttgcata aaattatacc ttagttttta 11160
   ctttgtatgt tacatgttag atttagagta tgaaaattag tagggaggat tattaacaaa 11220
   gaacagggca agaggagtag aattaaacct cttctaatac ctgtgcacaa gtaggctttt 11280
   cagaaactct acaaccccaa cataaactgg atagttagaa aagcacactc ccaaggaagg 11340
   cggttatgtt ttgcagtttg aatcagaaga atagagctat agcaatcttc attctatagt 11400
   aacattaaag agcctggttt atattatagc agtcattaag atttaaaaat ttacatcttg 11460
   ccgttcttct tactcacaga ttttcgagag gtaatgtaat gatcacacga ggtgagaatc 11520
   actgcctttt ataatgcgat taaatgcatg aacaaagttt ccaacaaata acagtaataa 11580
   aaagaaacat gtattagcac ttaataagcc aggtgctgta cgacgtgtgt tacatgcttt 11640
   caatccatga actggtaaac tggtactagt atctctattg gacatgtgag gaaaccaaat 11700
   ggagttgata aacagtagag ttaaaaatta ctcttcatat attatattgc ctcaatctca 11760
   cagacatctc tgctaccaaa agctatcata tctagactcg a                     11801
<210> 9
   <211> 19
   <212> DNA
   <213> Artificial Sequence
<220>
   <223> Description of Artificial Sequence:Probe/Primer
<400> 9
   tggcaacagg caagcagag 19
<210> 10
   <211> 21
   <212> DNA
   <213> Artificial Sequence
<220>
   <223> Description of Artificial Sequence:Probe/Primer
<400> 10
   ggccaaaata aaaccaaaca t 21
<210> 11
   <211> 24
   <212> DNA
   <213> Artificial Sequence
<220>
   <223> Description of Artificial Sequence:Probe/Primer
<400> 11
   gcaaatatga tttaaagata caac 24
<210> 12
   <211> 25
   <212> DNA
   <213> Artificial Sequence
<220>
   <223> Description of Artificial Sequence:Probe/Primer
<400> 12
   ggttgtatct ttaaatcata tttgc 25
<210> 13
   <211> 27
   <212> DNA
   <213> Artificial Sequence
<220>
   <223> Description of Artificial Sequence:Probe/Primer
<400> 13
   actgtctttt catatatttc tcaatgc 27
<210> 14
   <211> 24
   <212> DNA
   <213> Artificial Sequence
<220>
   <223> Description of Artificial Sequence:Probe/Primer
<400> 14
   aagtagtaat tttaaacatg ggac 24
<210> 15
   <211> 21
   <212> DNA
   <213> Artificial Sequence
<220>
   <223> Description of Artificial Sequence:Probe/Primer
<400> 15
   tttttcaatt aggcagcaac c 21
<210> 16
   <211> 25
   <212> DNA
   <213> Artificial Sequence
<220>
   <223> Description of Artificial Sequence:Probe/Primer
<400> 16
   gaattgtctt tgtgattgtt tttag 25
<210> 17
   <211> 26
   <212> DNA
   <213> Artificial Sequence
<220>
   <223> Description of Artificial Sequence:Probe/Primer
<400> 17
   caattcacaa agacaattca gttaag 26
<210> 18
   <211> 23
   <212> DNA
   <213> Artificial Sequence
<220>
   <223> Description of Artificial Sequence:Probe/Primer
<400> 18
   acaattagac aatgtccagc tga 23
<210> 19
   <211> 24
   <212> DNA
   <213> Artificial Sequence
<220>
   <223> Description of Artificial Sequence:Probe/Primer
<400> 19
   cttcggctga tatcatgaag tgtc 24
<210> 20
   <211> 23
   <212> DNA
   <213> Artificial Sequence
<220>
   <223> Description of Artificial Sequence:Probe/Primer
<400> 20
   aaccttttgc cctatgccgt aac 23
<210> 21
   <211> 22
   <212> DNA
   <213> Artificial Sequence
<220>
   <223> Description of Artificial Sequence:Probe/Primer
<400> 21
   gagactccca acctgatgat gt 22
<210> 22
   <211> 20
   <212> DNA
   <213> Artificial Sequence
<220>
   <223> Description of Artificial Sequence:Probe/Primer
<400> 22
   ggtcacgttg agtcccagtg 20

## Description

The present invention relates to nucleic acids, as defined in the claims, that are expressed in prostate tissue. More particularly, the present invention relates to the first of a family of novel, androgen-regulated, prostate-specific genes, PCGEM1, that is over-expressed in prostate cancer, and methods of using the PCGEM1 sequence and fragments thereof to measure the hormone responsiveness of prostate cancer cells and to detect or diagnose prostate cancer and other prostate related diseases.

Prostate cancer is the most common solid tumor in American men (1). The wide spectrum of biologic behavior (2) exhibited by prostatic neoplasms poses a difficult problem in predicting the clinical course for the individual patient (3, 4). Public awareness of prostate specific antigen (PSA) screening efforts has led to an increased diagnosis of prostate cancer. The increased diagnosis and greater number of patients presenting with prostate cancer has resulted in wider use of radical prostatectomy for localized disease (5). Accompanying the rise in surgical intervention is the frustrating realization of the inability to predict organ-confined disease and clinical outcome for a given patient (5,6). Traditional prognostic markers, such as grade, clinical stage, and pretreatment PSA have limited prognostic value for individual men. There is clearly a need to recognize and develop molecular and genetic biomarkers to improve prognostication and the management of patients with clinically localized prostate cancer. As with other common human neoplasia (7), the search for molecular and genetic biomarkers to better define the genesis and progression of prostate cancer is the key focus for cancer research investigations worldwide.

The new wave of research addressing molecular genetic alterations in prostate cancer is primarily due to increased awareness of this disease and the development of newer molecular technologies. The search for the precursor of prostatic adenocarcinoma has focused largely on the spectrum of microscopic changes referred to as "prostatic intraepithelial neoplasia" (PIN). Bostwick defines this spectrum as a histopathologic continuum that culminates in high grade PIN and early invasive cancer (8). The morphologic and molecular changes include the progressive disruption of the basal cell-layer, changes in the expression of differentiation markers of the prostatic secretory epithelial cells, nuclear and nucleolar abnormalities, increased cell proliferation, DNA content alterations, and chromosomal and allelic losses (8, 9). These molecular and genetic biomarkers, particularly their progressive gain or loss, can be followed to trace the etiology of prostate carcinogenesis. Foremost among these biomarkers would be the molecular and genetic markers associated with histological phenotypes in transition between normal prostatic epithelium and cancer. Most studies so far seem to agree that PIN and prostatic adenocarcinoma cells have a lot in common with each other. The invasive carcinoma more often reflects a magnification of some of the events already manifest in PIN.

Early detection of prostate cancer is possible today because of the widely propagated and recommended blood PSA test that provides a warning signal for prostate cancer if high levels of serum PSA are detected. However, when used alone, PSA is not sufficiently sensitive or specific to be considered an ideal tool for the early detection or staging of prostate cancer (10). Combining PSA levels with clinical staging and Gleason scores is more predictive of the pathological stage of localized prostate cancer (11). In addition, new molecular techniques are being used for improved molecular staging of prostate cancer (12, 13). For instance, reverse transcriptase - polymerase chain reaction (RT-PCR) can measure PSA of circulating prostate cells in blood and bone marrow of prostate cancer patients.

Despite new molecular techniques, however, as many as 25 percent of men with prostate cancer will have normal PSA levels - usually defined as those equal to or below 4 nanograms per milliliter of blood (14). In addition, more than 50 percent of the men with higher PSA levels are actually cancer free (14). Thus, PSA is not an ideal screening tool for prostate cancer. More reliable tumor-specific biomarkers are needed that can distinguish between normal and hyperplastic epithelium, and the preneoplastic and neoplastic stages of prostate cancer.

Identification and characterization of genetic alterations defining prostate cancer onset and progression is important in understanding the biology and clinical course of the disease. The currently available TNM staging system assigns the original primary tumor (T) to one of four stages (14). The first stage, T1, indicates that the tumor is microscopic and cannot be felt on rectal examination. T2 refers to tumors that are palpable but fully contained within the prostate gland. A T3 designation indicates the cancer has spread beyond the prostate into surrounding connective tissue or has invaded the neighboring seminal vesicles. T4 cancer has spread even further. The TNM staging system also assesses whether the cancer has metastasized to the pelvic lymph nodes (N) or beyond (M). Metastatic tumors result when cancer cells break away from the original tumor, circulate through the blood or lymph, and proliferate at distant sites in the body.

Recent studies of metastatic prostate cancer have shown a significant heterogeneity of allelic losses of different chromosome regions between multiple cancer foci (21-23). These studies have also documented that the metastatic lesion can arise from cancer foci other than dominant tumors (22). Therefore, it is critical to understand the molecular changes which define the prostate cancer metastasis especially when prostate cancer is increasingly detected in early stages (15-21).

Moreover, the multifocal nature of prostate cancer needs to be considered (22-23) when analyzing biomarkers that may have potential to predict tumor progression or metastasis. Approximately 50-60% of patients treated with radical prostatectomy for localized prostate carcinomas are found to have microscopic disease that is not organ confined, and a significant portion of these patients relapse (24). Utilizing biostatistical modeling of traditional and genetic biomarkers such as p53 and bcl-2, Bauer et al. (25-26) were able to identify, patients at risk of cancer recurrence after surgery. Thus, there is clearly a need to develop biomarkers defining various stages of the prostate cancer progression.

Another significant aspect of prostate cancer is the key role that androgens play in the development of both the normal prostate and prostate cancer. Androgen ablation, also referred to as "hormonal therapy," is a common treatment for prostate cancer, particularly in patients with metastatic disease (14). Hormonal therapy aims to inhibit the body from making androgens or to block the activity of androgen. One way to block androgen activity involves blocking the androgen receptor; however, that blockage is often only successful initially. For example, 70-80% of patients with advanced disease exhibit an initial subjective response to hormonal therapy, but most tumors progress to an androgen-independent state within two years (16). One mechanism proposed for the progression to an androgen-independent state involves constitutive activation of the androgen signaling pathway, which could arise from structural changes in the androgen receptor protein (16).

As indicated above, the genesis and progression of cancer cells involve multiple genetic alterations as well as a complex interaction of several gene products. Thus, various strategies are required to fully understand the molecular genetic alterations in a specific type of cancer. In the past, most molecular biology studies had focused on mutations of cellular proto-oncogenes and tumor suppressor genes (TSGs) associated with prostate cancer (7). Recently, however, there has been an increasing shift toward the analysis of "expression genetics" in human cancer (27-31), *i.e*., the under-expression or over-expression of cancer-specific genes. This shift addresses limitations of the previous approaches including: 1) labor intensive technology involved in identifying mutated genes that are associated with human cancer; 2) the limitations of experimental models with a bias toward identification of only certain classes of genes, *e.g*., identification of mutant *ras* genes by transfection of human tumor DNAs utilizing NIH3T3 cells; and 3) the recognition that the human cancer associated genes identified so far do not account for the diversity of cancer phenotypes.

A number of studies are now addressing the alterations of prostate cancer-associated gene expression in patient specimens (32-36). It is inevitable that more reports on these lines are to follow.

Thus, despite the growing body of knowledge regarding prostate cancer, there is still a need in the art to uncover the identity and function of the genes involved in prostate cancer pathogenesis. There is also a need for reagents and assays to accurately detect cancerous cells, to define various stages of prostate cancer progression, to identify and characterize genetic alterations defining prostate cancer onset and progression, to detect micro-metastasis of prostate cancer, and to treat and prevent prostate cancer.

EMBL Acc. No. AC003046, 8 Nov. 1997, provides homo sapiens Xp22 PACs RPC11-263P4 and RPC11-164K3.

Proceedings of the American Ass for Cancer Res. Ann. 40: 37, March 1999, describes the structure and expression of a prostate specific gene, PC-GEM1.

EMBL Acc. No AC013401, 11 Nov. 1999, provides homo sapiens chromosome 5 chromosome 5 clone RP11-98N11 (unordered pieces).

EMBL Acc. No. AF099810, 28 Oct. 1998, provides the homo sapiens neurexin III-alpha gene (chromosome 14 from 14q24.3-14q 32).

EMBL Acc. No. AC006925, 11 Mar. 1999, provides homo sapiens chromosome 17, clone hRPK.81_I_9.

WO 99/00498 describes a human NK-3 related prostate specific gene-1.

Bussemakers M.J.G. et al., Urological Research, Vol.25, No.1, pg 76, 1 Feb 1997, describes a prostate-specific marker, strongly overexpressed in prostatic tumours.

Wang Zhou et al., PNAS USA, Vol. 94, No.24, pgs 12999-13004, 25 Nov 1997, describes genes regulated by androgen in the rat ventral prostate.

WO 95/19434 describes a tissue-specific enhancer active in the prostate.

The present invention relates to the identification and characterization of a novel gene, the first of a family of genes, designated PCGEM1, for Prostate Cancer Gene Expression Marker 1. PCGEM1 is specific to prostate tissue, is androgen-regulated, and appears to be over-expressed in prostate cancer. More recent studies associate PCGEM1 cDNA with promoting cell growth. The invention provides the isolated nucleotide sequence of PCGEM 1 or fragments thereof and nucleic acid sequences that hybridize to PCGEM1, as defined in the claims. These sequences have utility, for example, as markers of prostate cancer and other prostate related diseases, and as targets for therapeutic intervention in prostate cancer and other prostate related diseases. The invention further provides a vector that directs the expression of PCGEM1, and a host cell transfected or transduced with this vector, as defined in the claims.

In another embodiment, the invention provides a method of detecting prostate cancer cells in a biological sample, as defined in the claims, such as by using nucleic acid amplification techniques with primers and probes selected to bind specifically to the PCGEM1 sequence. The invention further comprises a method of identifying an androgen responsive cell line, and a method of measuring responsiveness of a cell line to hormone-ablation therapy, as defined in the claims.

The specification also describes an isolated polypeptide encoded by the PCGEM1 gene or a fragment thereof, and antibodies generated against the PCGEM1 polypeptide, peptides, or portions thereof, which can be used to detect, treat, and prevent prostate cancer.

Thus, according to a first aspect of the invention, there is provided an isolated nucleic acid molecule selected from the group consisting of:
(a) the polynucleotide sequence consisting SEQ ID NO:1 or SEQ ID NO:2;
(b) the complement of (a); and
(c) a nucleic acid sequence consisting of SEQ ID NO:4, SEQ ID NO:6, SEQ ID NO:7, SEQ ID NO:9, SEQ ID NO:10, SEQ ID NO:11, SEQ ID NO:12, SEQ ID NO:13, SEQ ID NO:14, SEQ 113 NO:15, SEQ ID NO: 16, SEQ ID NO: 17, SEQ ID NO: 18, SEQ ID NO:19, SEQ ID NO:20, SEQ ID NO:21 or SEQ ID NO:22.

In another aspect of the invention there is provided an *in vitro* method of detecting prostate cancer in a patient, the method comprising:
(a) detecting a mRNA sequence in a biological sample from the patient, by hybridising the mRNA sequence to SEQ ID NO:1 or SEQ ID NO:2 under conditions of high stringency; and
(b) correlating the amount of the mRNA sequence in the sample with the presence of prostate cancer in the patient.

The invention also provides an in vitro method of identifying an androgen-responsive cell line, the method comprising:
(a) incubating a cell line suspected of being androgen responsive with an androgen; and
(b) detecting a mRNA sequence in the cell line, by hybridising the mRNA sequence to SEQ ID NO:1 or SEQ ID NO:2 under conditions of high stringency;
wherein an increase in the mRNA sequence, as compared to an untreated cell line, correlates with the cell line being androgen responsive.

In another aspect there is provided an in vitro method of measuring the responsiveness of a prostate tissue to hormone-ablation therapy, as defined in the claims.

In further embodiments of the invention there are also provided an isolated nucleic acid molecule consisting of a fragment of SEQ ID NO: 1, wherein the fragment comprises at least 20 contiguous nucleotides of SEQ ID NO:1, as defined in the claims.

The invention will now be described in more detail, with particular reference to the accompanying drawings.

### BRIEF DESCRIPTION OF THE DRAWINGS

Figure 1 depicts the scheme for the identification of differentially expressed genes in prostate tumor and normal tissues.
Figure 2 depicts a differential display pattern of mRNA obtained from matched tumor and normal tissues of a prostate cancer patient. Arrows indicate differentially expressed cDNAs.

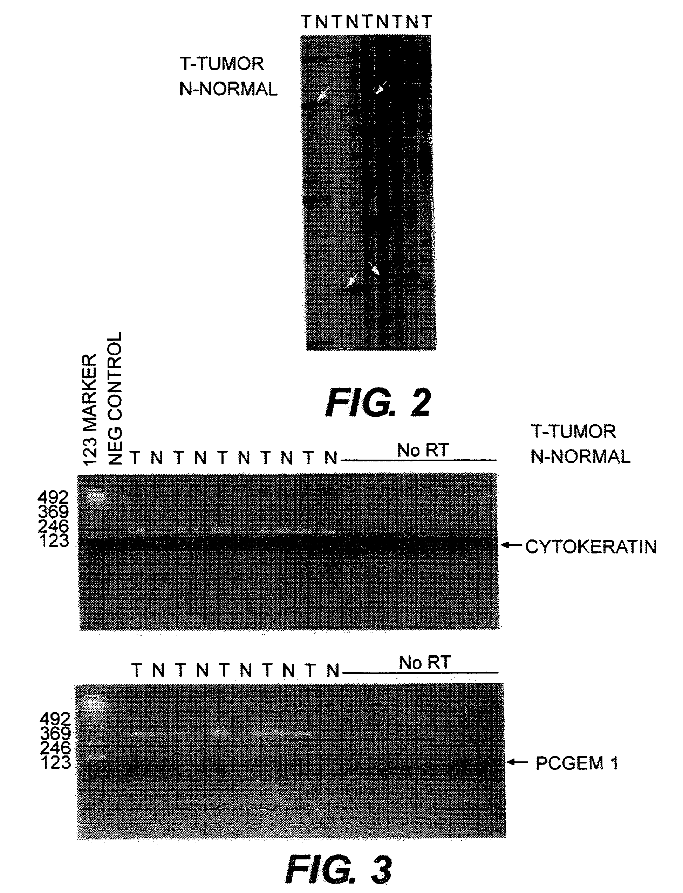

Figure 3 depicts the analysis of PCGEM1 expression in primary prostate cancers. PCGEM1 specific PCR primers were designed for RT-PCR analysis. Microdissected tissue derived 100 nanograms of genomic DNA-free RNA was used for RT-PCR. The PCR conditions were optimized to be within the logarithmic phase of amplication for all primers used. Epitheal cell associated cytokeratin-18 was used as an internal control The PCR was performed using Amplitaq Gold. PCR cycles included: 95°C for 10 minutes, 1 cycle followed by 95°C for 30 seconds, 55°C for 30 seconds, 72°C for 30 seconds, 42 cycles (PCGEM1) or 35 cycles (cytokertain-18) and 72°C for 5 minutes final extension followed by 4°C chilling. Three independent experiments showed the same results.
Figure 4 depicts the expression pattern of PCCEM1 in prostate cancer cell lines. Prostate cancer cell lines: LNCaP, DU145, PC-3, DuPro and CPDR-1 were analyzed for PCGEM1 expression utilizing RT-PCR conditions as described in Figure 3.
Figure 5a depicts the androgen regulation of PCOEM1 expression in LNCaP cells, as measured by reverse transcriptase PCR. LNCaP cells were cultured in RPMI medium containing 10% charcoal stripped fetal bovine serum for 4 days followed by treatment with synthetic androgen: R1881 for 12 hours and 24 hours at 0.1 nanomolar and 10 nanomolar concentrations. Ply-A+RNA from treated and untreated cells were analyzed for PCGEM1 expression analysis by RT-PCR as described in Figure 3.
Figure 5b depicts the androgen regulation of PCGEM1 expression in LNCaP cells, as measured by Northern blot hybridization. LNCaP cells were cultured in RPMI medium containing 10% charcoal stripped fetal bovine serum for 4 days followed by treatment with synthetic androgen: R1881 for 24 hours at 0.1 nanomolar concentration. Poly-A+RNA from treated and untreated cells were analyzed for PCGEM1 expression by Northern blot hybridization.
Figure 6a depicts the prostate tissue specific expression pattern of PCGEM1. Multiple tissue Northern blots (Clontech, CA) were probed with a 530bp PCGEM1 cDNA probe. Prostate tissue specific expression of a 1.7kb mRNA band was detected. A recent independent experiment confirmed these results.
Figure 6b depicts a RNA master blot (Clontech, CA) showing the prostate tissue specificity of PCGEM1.
Figure 7A depicts the chromosomal localization of PCGEM1 by fluorescent in situ hybridization analysis. FISH analysis was done using genomic DNA from PCGEM1 Bac clone. The DNA was nick translated using biotin labeled kit or digoxinin labeled kit. The metaphase chromosome preparation was hybridized and analyzed.
Figure 7B depicts a DAPI counter-stained chromosome 2 (left), an inverted DAPI stained chromosome 2 shown as G-bands (center), and an ideogram of chromosome 2 showing the localization of the signal to band 2q32(bar).
Figure 8 depicts a cDNA sequence of PCGEM1 (SEQ ID NO:1). Full length cDNA sequence of PCGEM1 was obtained by 5 prime and 3 prime RACE Marathon ready cDNA kit (Clontech, CA) and screening of normal human prostate cDNA library (Clontech, CA) using original PCGEM1 fragment as probe. The DNA sequencing was performed on ABI-310 sequence analyzer.
Figure 9 depicts an additional cDNA sequence of PCGEMl1 (SEQ ID NO:2). Full.length cDNA sequence of PCGEM1 was obtained by a 5 prime and 3 prime RACE Marathon ready cDNA kit (Clontech, CA) and screening of normal human Prostate cDNA library (Clontech, CA) using original PCGEM1 fragment as probe. The DNA sequencing was performed on ABI-310 sequence analyzer.
Figure 10 depicts the colony formation of NIH3T3 cell lines expressing various PCGEM1 constructs. PCGEM1 expression vector in sense and antisense orientations [PCDNA3.1/Hygro(+/-) from (Invitrogen, CA)] were transfected into NIH3T3 cells. Hygromycin resistant colonies were counted 2-3 weeks after staining with crystal violet

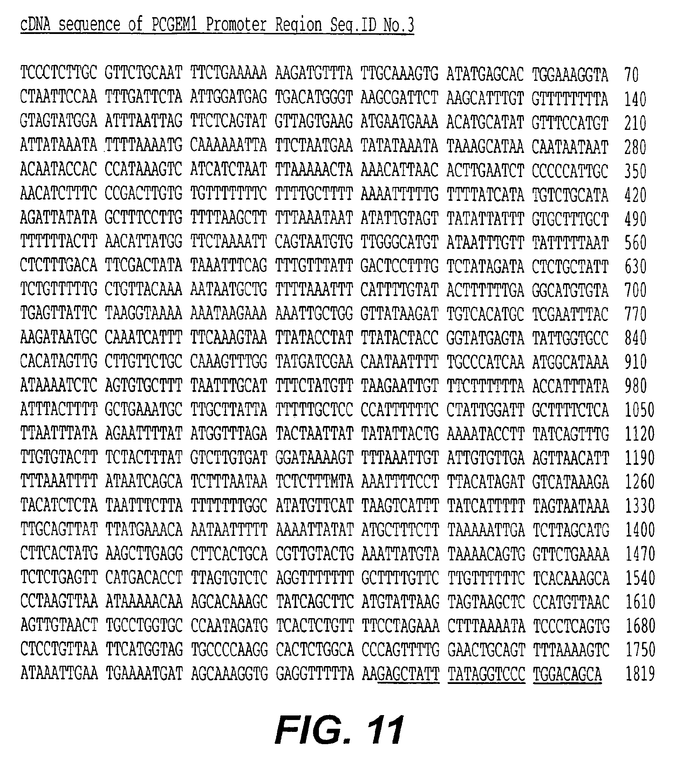

Figure 11 depicts the cDNA sequence of the promoter region of PCGEM1 SEQ ID NO:3. The promoter region of PCGEM1 gene was obtained from human placenta genomic library (Stratagene, CA) by PCR using a PCGEM1 specific primer (underlined sequence) and a T7 promoter primer. The PCR product was cloned into TA-Cloning vector (Invitrogen, CA) and sequenced by 310 DNA sequence analyzer (Perkin-Elmer, CA). The sequence was further verified by direct sequencing of PCR product from placenta DNA. The triangle indicates the putative transcription start site.
Figure 12 depicts the cDNA of a probe, designated SEQ ID NO:4.
Figure 13 depicts the cDNAs of primers 1-3, designated SEQ ID NOs:5-7, respectively.
Figure 14 depicts the genomic DNA sequence of PCGEM1, designated SEQ ID NO:8.
Figure 15 depicts the structure of the PCGEM1 transcription unit.
Figure 16 depicts a graph of the hypothetical coding capacity of PCGEM1.
Figure 17 depicts a representative example of *in situ* hybridization results showing PCGEM1 expression in normal and tumor areas of prostate cancer tissues.

The present invention relates to PCGEM1, the first of a family of genes, and its related nucleic acids, as defined in the claims, for use in the detection, of prostate cancer (*e.g*., prostatic intraepithelial neoplasia (PIN), adenocarcinomas, nodular hyperplasia, and large duct carcinomas) and prostate related diseases (e,g., benign prostatic hyperplasia), as recited in the claims.

Although we do not wish to be limited by any theory or hypothesis, preliminary data suggest that the PCGEM1 nucleotide sequence may be related to a family of non-coding poly A+RNA that may be implicated in processes relating to growth and embryonic development (40-44). Evidence presented herein supports this hypothesis. Alternatively, PCGEM1 cDNA may encode a small peptide.

In a particular embodiment, the invention relates to certain isolated nucleotide sequences that are substantially free from contaminating endogenous material, as defined in the claims. A "nucleotide-sequence" refers to a polynucleotide molecule in the form of a separate fragment or as a component of a larger nucleic acid construct. The nucleic acid molecule has been derived from DNA or RNA isolated at least once in substantially pure form and in a quantity or concentration enabling identification, manipulation, and recovery of its component nucleotide sequences by standard biochemical methods (such as those outlined in Sambrook et al., Molecular Cloning: A Laboratory Manual, 2nd ed., Cold Spring Harbor Laboratory, Cold Spring Harbor, NY (1989)).

Nucleic acid molecules include DNA in both single-stranded and double-stranded form, as well as the RNA complement thereof. DNA includes, for example, cDNA, genomic DNA, chemically synthesized DNA, DNA amplified by PCR, and combinations thereof Genomic DNA may be isolated by conventional techniques, *e.g*., using the cDNA of SEQ ID NO: 1, SEQ ID NO:2, or suitable fragments thereof as a probe.

The DNA molecules of the invention include fun length genes as well as polynucleotides and fragments thereof, as defined in the claims. The full length gene may include the N-terminal signal peptide. Although a non-coding role of PCGEM1 appears likely, the possibility of a protein product cannot presently be ruled out. Therefore, other embodiments may include DNA encoding a soluble form, *e.g*., encoding the extracellular domain of the protein, either with or without the signal peptide.

The nucleic acids of the invention are preferentially derived from human sources, but the specification describes those derived from non-human species, as well.

### Preferred Sequences

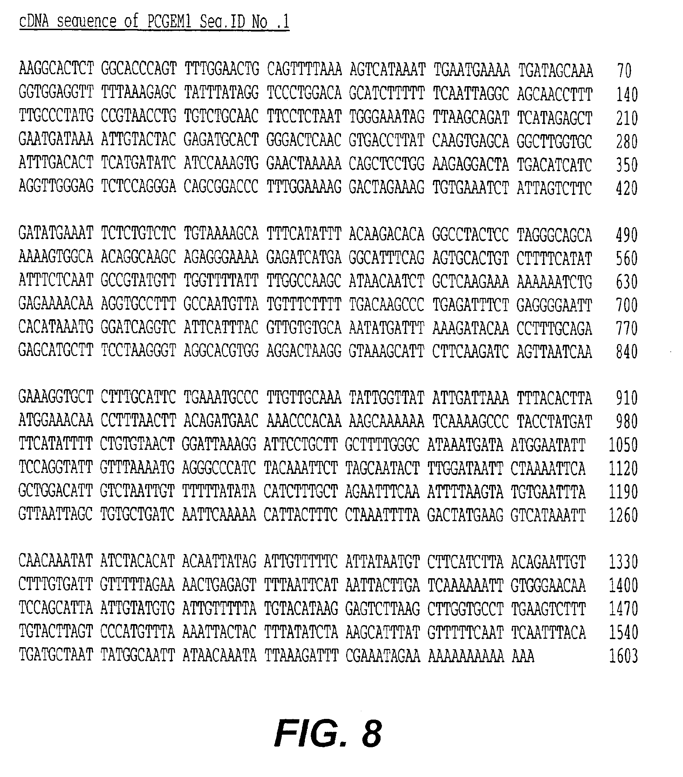

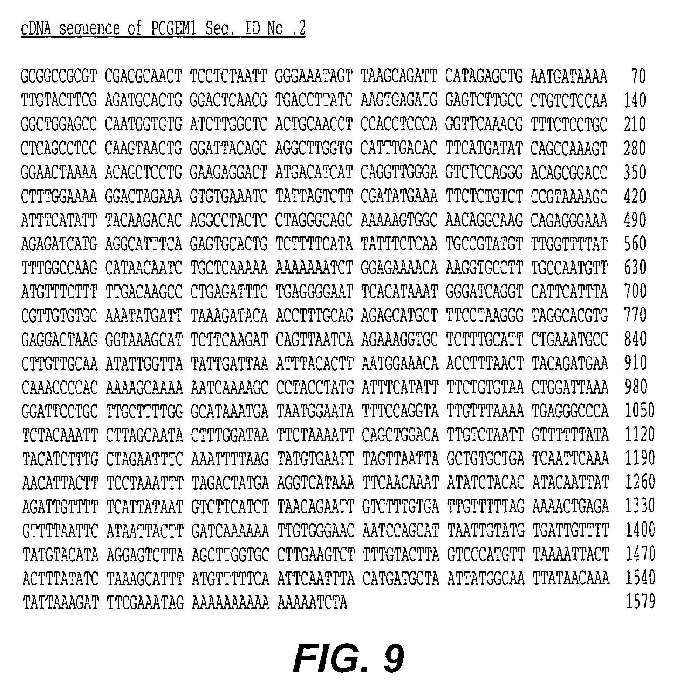

Particularly preferred nucleotide sequences of the invention are SEQ ID NO:1 and SEQ ID NO:2, as set forth in Figures 8 and 9, respectively. Two cDNA clones having the nucleotide sequences of SEQ ID NO:1 and SEQ ID NO:2, and the genomic DNA having the nucleotide sequence of SEQ ID NO: 8, were isolated as described in Example 2.

Thus, in a particular embodiment, this invention provides an isolated nucleic acid molecule selected from the group consisting of (a) the polynucleotide sequence consisting of SEQ ID NO:1 or SEQ ID NO:2 (b) the complement of (a) and (c) a nucleic acid sequence as defined in the claims.

As used herein, conditions of moderate stringency can be readily determined by those having ordinary skill in the art based on, for example, the length of the DNA. The basic conditions are set forth by Sambrook et al. Molecular Cloning: A Laboratory Manual, 2d ed. Vol. 1, pp. 1.101-104, Cold Spring Harbor Laboratory Press, (1989), and include use of a prewashing solution for the nitrocellulose filters of about 5X SSC, about 0.5% SDS, and about 1.0 mM EDTA (pH 8.0), hybridization conditions of about 50% formamide, about 6X SSC at about 42°C (or other similar hybridization solution, such as Stark's solution, in about 50% formamide at about 42°C), and washing conditions of about 60°C, about 0.5X SSC, and about 0.1% SDS. Conditions of high stringency can also be readily determined by the skilled artisan based on, for example, the length of the DNA. Generally, such conditions are defined as hybridization conditions as above, and with washing at approximately 68°C, about 0.2X SSC, and about 0.1% SDS. The skilled artisan will recognize that the temperature and wash solution salt concentration can be adjusted as necessary according to factors such as the length of the probe.

### Additional Sequences

Due to the known degeneracy of the genetic code, wherein more than one codon can encode the same amino acid, a DNA sequence can vary from that shown in SEQ ID NO:1 or SEQ ID NO:2, and still encode PCGEM1. Such variant DNA sequences can result from silent mutations (*e.g*., occurring during PCR amplification), or can be the product of deliberate mutagenesis of a native sequence.

The invention thus relates to isolated DNA sequences of the invention selected from: (a) DNA consisting of the nucleotide sequence ofSEQ ID NO:1 or SEQ ID NO:2; (b) the complement thereof; or (c) a fragment thereof as defined in the claims. Such sequences are preferably provided and/or constructed in the form of an open reading frame uninterrupted by internal non-translated sequences, or introns, that are typically present in eukaryotic genes. Sequences of non-translated DNA can be present 5' or 3' from an open reading frame, where the same do not interfere with manipulation or expression of the coding region. Of course, should PCCEM1 encode a polypeptide, polypeptides are encoded by such DNA sequences.

Percent identity may be determined by visual inspection and mathematical calculation. Alternatively, percent identity of two nucleic acid sequences may be determined by comparing sequence information using the GAP computer program, version 6.0 described by Devereux et al. (Nucl. Acids Res. 12:387,1984) and available from the University of Wisconsin Genetics Computer Group (UWGCG). The preferred default parameters for the GAP program include: (1) a unary comparison matrix (containing a value of 1 for identities and 0 for non-identities) for nucleotides, and the weighted comparison matrix of Gribskov and Burgess, Nucl. Acids Res. 14:6745, 1986, as described by Schwartz and Dayhoff, eds., Atlas of Protein Sequence and Structure, National Biomedical Research Foundation, pp. 353-358, 1979; (2) a penalty of 3.0 for each gap and an additional 0.10 penalty for each symbol in each gap; and (3) no penalty for end gaps. Other programs used by one skilled in the art of sequence comparison may also be used.

Isolated nucleic acids may be useful in the production of polypeptides. Such polypeptides may be prepared by any of a number of conventional techniques. A DNA sequence of this invention or desired fragment thereof may be subcloned into an expression vector for production of the polypeptide or fragment. The DNA sequence advantageously is fused to a sequence encoding a suitable leader or signal peptide. Alternatively, the desired fragment may be chemically synthesized using known techniques. DNA fragments also may be produced by restriction endonuclease digestion of a full length cloned DNA sequence, and isolated by electrophoresis on agarose gels. If necessary, oligonucleotides that reconstruct the 5' or 3' terminus to a desired point may be ligated to a DNA fragment generated by restriction enzyme digestion. Such oligonucleotides may additionally contain a restriction endonuclease cleavage site upstream of the desired coding sequence, and position an initiation codon (ATG) at the N-terminus of the coding sequence.

The well-known polymerase chain reaction (PCR) procedure also may be employed to isolate and amplify a DNA sequence encoding a desired protein fragment. Oligonucleotides that define the desired termini of the DNA fragment are employed as 5' and 3' primers. The oligonucleotides may additionally contain recognition sites for restriction endonucleases, to facilitate insertion of the amplified DNA fragment into an expression vector. PCR techniques are described in Saiki et al., Science 239:487 (1988); Recombinant DNA Methodology, Wu et al., eds., Academic Press, Inc., San Diego (1989), pp. 189-196; and PCR Protocols: A Guide to Methods and Applications, Innis et al., eds., Academic Press, Inc. (1990).

### USE OF PCGEM1 NUCLEIC ACID OR OLIGONUCLEOTIDES

In a particular embodiment, the invention relates to PCGEM1 nucleotide sequences isolated from human prostate cells, including two full length cDNAs: SEQ ID NO:1 (Figure 8) and SEQ ID NO:2 (Figure 9), and fragments thereof as defined in the claims. The nucleic acids of the invention, including DNA, RNA, mRNA and oligonucleotides thereof, are useful in a variety of applications in the detection, diagnosis, prognosis, and treatment of prostate cancer. Examples of applications envisaged include, but are not limited to:
- amplifying PCGEM1 sequences;
- detecting a PCGEM1-derived marker of prostate cancer by hybridization with an oligonucleotide probe;
- identifying chromosome 2;
- mapping genes to chromosome 2;
- identifying genes associated with certain diseases, syndromes, or other conditions associated with human chromosome 2;
- constructing vectors having PCGEM1 sequences;
- expressing vector-associated PCGEM1 sequences as RNA and protein;
- detecting defective genes in an individual;
- developing gene therapy;
- developing immunologic reagents corresponding to PCGEM1-encoded products; and
- treating prostate cancer using antibodies, antisense nucleic acids, or other inhibitors specific for PCGEM1 sequences.

### Detecting and Diagnosing Prostate Cancer

The present invention provides an in vitro method of detecting prostate cancer in a patient, which comprises (a) detecting PCGEM1 mRNA in a biological sample from the patient, as defined in the claims, and (b) correlating the amount of PCGEM1 mRNA in the sample with the presence of prostate cancer in the patient. In one embodiment, detecting PCGEM1 mRNA in a biological sample includes: (a) isolating am RNA from a biological sample from the patient, (b) amplifying a cDNA molecule comprising SEQ ID NO:1, or a fragment thereof; (c) incubating the amplified cDNA with a nucleic acid probe that hybridises to SEQ ID No:1 to form a duplex molecule that is both stable and selective; and (d) detecting hybridization between the amplified cDNA and the probe. The biological sample can be selected from the group consisting of blood, urine, and tissue, for example, from a biopsy. In a preferred embodiment, the biological sample is blood. This method is useful in both the initial diagnosis of prostate cancer, and the later prognosis of disease. This method allows for testing prostate tissue in a biopsy, and after removal of a cancerous prostate, continued monitoring of the blood for micrometastases.

According to this method of diagnosing and prognosticating prostate cancer in a patient, the amount of PCGEM1 mRNA in a biological sample from a patient is correlated with the presence of prostate cancer in the patient Those of ordinary skill in the art can readily assess the level of over-expression that is correlated with the presence of prostate cancer.

This invention also provides a method of identifying an androgen-responsive cell line, which comprises (a) incubating a cell line suspected of being androgen-responsive with an androgen; and (b) detecting a mRNA sequence in the cell line, by hybridising said mRNA sequence to SEQ ID NO: 1 or 2 under conditions of high stringency; wherein an increase in PCGEM1 mRNA, as compared to an untreated cell line, correlates with the cell line being androgen-responsive.

The invention further provides an in vitro method of measuring the responsiveness of a prostatic tissue to hormone-ablation therapy, which comprises measuring a mRNA sequence in the prostatic tissue following hormone-ablation therapy, by hybridising said mRNA sequence to SEQ ID NO: 1 or 2 under conditions of high stringency; wherein a decrease in PCGEM1 mRNA, as compared to an untreated cell line, correlates with the cell line responding to hormone-ablation therapy.

These nucleic acid molecules may be introduced into a recombinant vector, such as a plasmid, cosmid, or virus, which can be used to transfect or transduce a host cell. Nucleic acids may be combined with other DNA sequences, such as promoters, polyadenylation signals, restriction enzyme sites, multiple cloning, sites, and other coding sequences.

### Probes

Among the uses of nucleic acids of the invention is the use of fragments as defined in the claims as probes or primers. Examples of probes or primers of the invention include those of SEQ ID NO: 6, and SEQ ID NO: 7, as well as those disclosed in Table I.

**Table I**

| **Primer** | **Sequence (5'→3')** | **S/AS** | **Starting Base #** | **SEQ ID NO.** |
|---|---|---|---|---|
| p413 | TGGCAACAGGCAAGCAGAG | S | 510 | SEQ ID NO: 9 |
| p414 | GGCCAAAATAAAACCAAACAT | AS | 610 | SEQ ID NO: 10 |
| p489 | GCAAATATGATTTAAAGATACAAC | S | 752 | SEQ ID NO: 11 |
| p490 | GGTTGTATCTTTAAATCATATTTGC | AS | 776 | SEQ ID NO: 12 |
| p491 | ACTGTCTTTTCATATATTTCTCAATGC | S | 559 | SEQ ID NO: 13 |
| p517 | AAGTAGTAATTTTAAACATGGGAC | AS | 1516 | SEQ ID NO: 14 |
| p518 | TTTTTCAATTAGGCAGCAACC | S | 131 | SEQ ID NO: 15 |
| p519 | GAATTGTCTTTGTGATTGTTTTTAG | S | 1338 | SEQ ID NO: 16 |
| p560 | CAATTCACAAAGACAATTCAGTTAAG | AS | 1355 | SEQ ID NO: 17 |
| p561 | ACAATTAGACAATGTCCAGCTGA | AS | 1154 | SEQ ID NO: 18 |
| p562 | CTTTGGCTGATATCATGAAGTGTC | AS | 322 | SEQ ID NO: 19 |
| p623 | AACCTTTTGCCCTATGCCGTAAC | S | 148 | SEQ ID NO: 20 |
| p624 | GAGACTCCCAACCTGATGATGT | AS | 376 | SEQ ID NO: 21 |
| p839 | GGTCACGTTGAGTCCCAGTG | AS | 270 | SEQ ID NO: 22 |

| | | | | |
|---|---|---|---|---|
| S/AS indicates whether the primer is Sense or AntiSense Starting Base # indicates the starting base number with respect to the sequence of SEQ ID NO:1. | | | | |

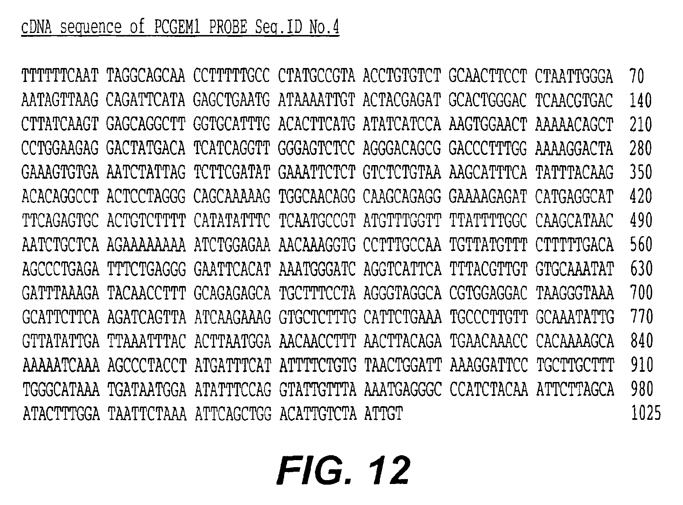

However, even larger probes may be used. For example, a particularly preferred probe is derived from PCGEM1 (SEQ ID NO: 1) and comprises nucleotides 116 to 1140 of that sequence. It has been designated SEQ ID NO: 4 and is set forth in Figure 12.

When a hybridization probe binds to a target sequence, it forms a duplex molecule that is both stable and selective. These nucleic acid molecules may be readily prepared, for example, by chemical synthesis or by recombinant techniques. A wide variety of methods are known in the art for detecting hybridization, including fluorescent, radioactive, or enzymatic means, or other ligands such as avidin/biotin.

In another aspect of the invention, these nucleic acid molecules may be introduced into a recombinant vector, such as a plasmid, cosmid, or virus, which can be used to transfect or transduce a host cell. The nucleic acids of the present invention may be combined with other DNA sequences, such as promoters, polyadenylation signals, restriction enzyme sites, multiple cloning sites, and other coding sequences. Probes based on the human DNA sequence of SEQ ID NO:1 or SEQ ID NO:2 may be used to screen cDNA libraries derived from other mammalian species, using conventional cross-species hybridization techniques.

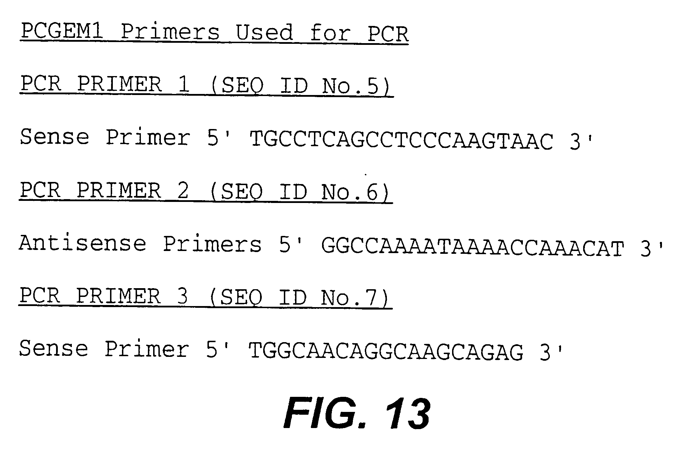

One can use the knowledge of the genetic code in combination with the sequences set forth herein to prepare sets of degenerate oligonucleotides. Such oligonucleotides are useful as primers, e.g., in polymerase chain reactions (PCR), whereby DNA fragments are isolated and amplified. Particularly preferred primers are set forth in Figures 13 and Table I and are designated SEQ ID NOS: 6-7 and 9-22, respectively. A particularly preferred primer pair is p518 (SEQ ID NO: 15) and p839 (SEQ ID NO: 22), which when used in PCR, preferentially amplifies mRNA, thereby avoiding less desirable cross-reactivity with genomic DNA.

### Chromosome Mapping

As set forth in Example 3, the PCGEM1 gene has been mapped by fluorescent in situ hybridization to the 2q32 region of chromosome 2 using a bacterial artificial chromosome (BAC) clone containing PCGEU1 genomic sequence. Thus, all or a portion of the nucleic acid molecule of SEQ ID NO:1 and SEQ ID NO:2 , including oligonucleotides, can be used by those skilled in the art using well-known techniques to identify human chromosome 2, and the specific locus thereof, that contains the PCGEM1 DNA. Useful techniques include, but are not limited to, using the nucleotide sequence of SEQ ID NO:1 or SEQ ID NO:2, or fragments thereof, including oligonucleotides, as a probe in various well-known techniques such as radiation hybrid mapping (high resolution), *in situ* hybridization to chromosome spreads (moderate resolution), and Southern blot hybridization to hybrid cell lines containing individual human chromosomes (low resolution).

For example, chromosomes can be mapped by radiation hybridization. First, PCR is performed using the Whitehead Institute/MIT Center for Genome Research Genebridge4 panel of 93 radiation hybrids

### (http://www-genome.wi.mit.edu/ftp/distribution/

human_STS_releases/july97/rhmap/genebridge4.html). Primers are used which lie within a putative exon of the gene of interest and which amplify a product from human genomic DNA, but do not amplify hamster genomic DNA. The results of the PCRs are converted into a data vector that is submitted to the Whitehead/MIT Radiation Mapping site on the internet (http://www-seq.wi.mit.edu). The data is scored and the chromosomal assignment and placement relative to known Sequence Tag Site (STS) markers on the radiation hybrid map is provided. (The following web site provides additional information about radiation hybrid mapping: http://www-genome.wi.mit.edu/ftp/distribution/human_STS_releases/july97/ 07-97.INTRO.html).

### Identifying Associated Diseases

As noted above, PCGEM1 has been mapped to the 2q32 region of chromosome 2. This region is associated with specific diseases, which include but are not limited to diabetes mellitus (insulin dependent), and T cell leukemia/lymphoma. Thus, the nucleic acids of SEQ ID NO: 1 or SEQ ID NO: 2, or fragments thereof, as defined in the claims, can be used by one skilled in the art using well-known techniques to analyze abnormalities associated with gene mapping to chromosome 2. This enables one to distinguish conditions in which this marker is rearranged or deleted. In addition, nucleotides of SEQ ID NO:1 or SEQ ID NO:2, or fragments thereof as defined in the claims, can be used as a positional marker to map other genes of unknown location.

The DNA may be used in developing treatments for any disorder mediated (directly or indirectly) by defective, or insufficient amounts of PCGEM1, including prostate cancer. Disclosure herein of native nucleotide sequences permits the detection of defective genes, and the replacement thereof with normal genes. Defective genes may be detected in *in vitro* diagnostic assays, and by comparison of a native nucleotide sequence disclosed herein with that of a gene derived from a person suspected of harboring a defect in this gene.

### Sense-Antisense

Other useful fragments of nucleic acids include antisense or sense oligonucleotides comprising a single-stranded nucleic acid sequence (either RNA or DNA) capable of binding to target mRNA (sense) or DNA (antisense) sequences. Antisense or sense oligonucleotides comprise a fragment of DNA (SEQ ID NO:1 or SEQ ID NO:2). Such a fragment generally comprises at least about 14 nucleotides, preferably from about 14 to about 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988).

The biologic activity of PCGEM1 in assay cells and the over expression of PCGEM1 in prostate cancer tissues suggest that elevated levels of PCGEM1 promote prostate cancer cell growth. Thus, the antisense oligonucleotides to PCGEM1 may be used to reduce the expression of PCGEM1 and, consequently, inhibit the growth of the cancer cells.

Binding of antisense or sense oligonucleotides to target nucleic acid sequences results in the formation of duplexes. The antisense oligonucleotides thus may be used to block expression of proteins or to inhibit the function of RNA. Antisense or sense oligonucleotides further comprise oligonucleotides having modified sugar-phosphodiester backbones (or other sugar linkages, such as those described in WO91/06629) and wherein such sugar linkages are resistant to endogenous nucleases. Such oligonucleotides with resistant sugar linkages are stable *in vivo* (*i.e.,* capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10448, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides. Such modifications may modify binding specificities of the antisense or sense oligonucleotide for the target nucleotide sequence.

Antisense or sense oligonucleotides may be introduced into a cell containing the target nucleic acid sequence by any gene transfer method, including, for example, lipofection, CaPO₄-mediated DNA transfection, electroporation, or by using gene transfer vectors such as Epstein-Barr virus or adenovirus.

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

### POLYPEPTIDES AND FRAGMENTS THEREOF

The specification describes polypeptides and fragments thereof in various forms, including those that are naturally occurring or produced through various techniques such as procedures involving recombinant DNA technology. Such forms include, but are not limited to, derivatives, variants, and oligomers, as well as fusion proteins or fragments thereof.

Thus, the polypeptides described herein include full length proteins encoded by the nucleic acid sequences set forth above. The polypeptides may be membrane bound or they may be secreted and thus soluble. The specification also describes the expression, isolation and purification of polypeptides and fragments accomplished by any suitable technique.

### EXAMPLE 1: Differential Gene Expression Analysis in Prostate Cancer

Using the differential display technique, we identified a novel gene that is over-expressed in prostate cancer cells. Differential display provides a method to separate and clone individual messenger RNAs by means of the polymerase chain reaction, as described in Liang et al., Science, 257:967-71 (1992), which is hereby incorporated by reference. Briefly, the method entails using two groups of oligonucleotide primers. One group is designed to recognize the polyadenylate tail of messenger RNAs. The other group contains primers that are short and arbitrary in sequence and anneal to positions in the messenger RNA randomly distributed from the polyadenylate tail. Products amplified with these primers can be differentiated on a sequencing gel based on their size. If different cell populations are amplified with the same groups of primers, one can compare the amplification products to identify differentially expressed RNA sequences.

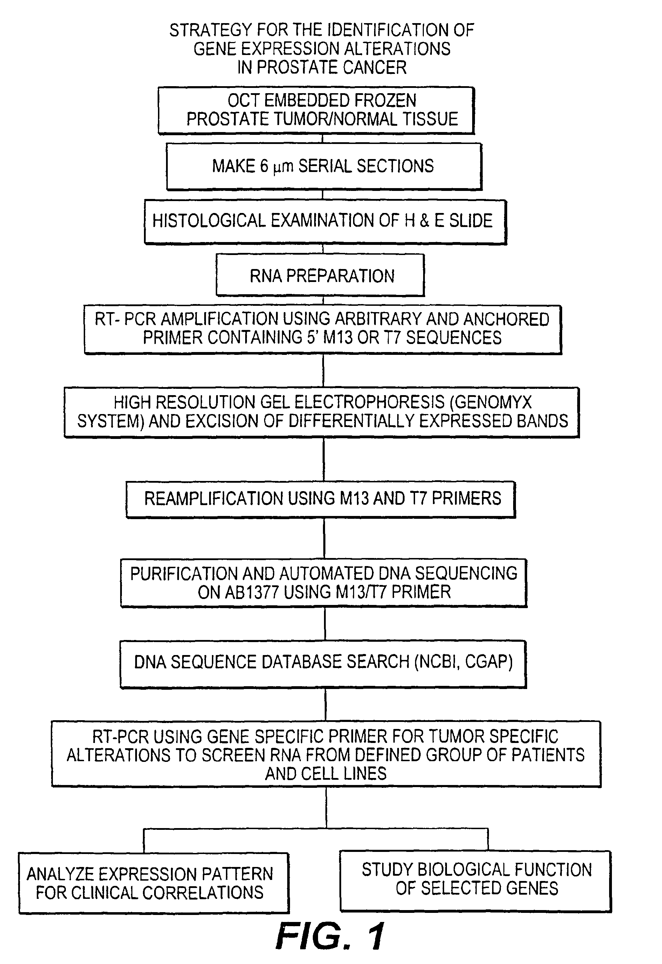

Differential display ("DD") kits from Genomyx (Foster City, California) were used to analyze differential gene expression. The steps of the differential display technique are summarized in Figure 1. Histologically well defined matched tumor and normal prostate tissue sections containing approximately similar proportions of epithelial cells were chosen from individual prostate cancer patients.

Genomic DNA-free total RNA was extracted from this enriched pool of cells using RNAzol B (Tel-Test, Inc., Friendswood, TX) according to manufacturer's protocol. The epithelial nature of the RNA source was further confirmed using cytokeratin 18 expression (45) in reverse transcriptase-polymerase chain reaction (RT-PCR) assays. Using arbitrary and anchored primers containing 5' M13 or T7 sequences (obtained from Biomedical Instrumentation Center, Uniformed Services University of the Health Sciences, Bethesda), the isolated DNA-free total RNA was amplified by RT-PCR which was performed using ten anchored antisense primers and four arbitrary sense primers according to the protocol provided by Hieroglyph™ RNA Profile Kit 1 (Genomyx Corporation, CA). The cDNA fragments produced by the RT-PCR assay were analyzed by high resolution gel electrophoresis, carried out by using Genomyx™ LR DNA sequencer and LR-Optimized™ HR-1000™ gel formulations (Genomyx Corporation, CA).

A partial DD screening of normal/tumor tissues revealed 30 differentially expressed cDNA fragments, with 53% showing reduced or no expression in tumor RNA specimens and 47% showing over expression in tumor RNA specimen (Figure 2). These cDNAs were excised from the DD gels, reamplified using T7 and M13 primers and the RT PCR conditions recommended in Hieroglyph™ RNA Profile Kit-1 (Genomyx Corp., CA), and sequenced. The inclusion of T7 and M 13 sequencing primers in the DD primers allowed rapid sequencing and orientation of cDNAs (Figure 1).

All the reamplified cDNA fragments were purified by Centricon-c-100 system (Amicon, USA). The purified fragments were sequenced by cycle sequencing and DNA sequence determination using an ABI 377 DNA sequencer. Isolated sequences were analyzed for sequence homology with known sequences by running searches through publicly available DNA sequence databases, including the National Center for Biotechnology Information and the Cancer Genome Anatomy Project. Approximately two-thirds of these cDNA sequences exhibited homology to previously described DNA sequences/genes e.g., ribosomal proteins, mitochondrial DNA sequences, growth factor receptors, and genes involved in maintaining the redox state in cells. About one-third of the cDNAs represented novel sequences, which did not exhibit similarity to the sequences available in publicly available databases. The PCGEM1 fragment, obtained from the initial differential display screening represents a 530 base pair (nucleotides 410 to 940 of SEQ ID NO: 1) cDNA sequence which, in initial searches, did not exhibit any significant homology with sequences in the publicly available databases. Later searching of the high throughput genome sequence (HTGS) database revealed perfect homology to a chromosome 2 derived uncharacterized, unfinished genomic sequence (accession # AC 013401).

### EXAMPLE 2: Characterization of Full Length PCGEM1 cDNA Sequence

The full length of PCGEM1 was obtained by 5' and 3' RACE/PCR from the original 530 bp DD product (nucleotides 410 to 940 of PCGEM1 DNA SEQ ID NO:1) using a normal prostate cDNA library in lambda phage (Clontech, CA). The RACE/PCR products were directly sequenced. Lasergene and MacVector DNA analysis software were used to analyze DNA sequences and to define open reading frame regions. We also used the original DD product to screen a normal prostate cDNA library. Three overlapping cDNA clones were identified.

Sequencing of the cDNA clones was performed on an ABI-310 sequence analyzer and a new dRhodamine cycle sequencing kit (PE-Applied Biosystem, CA). The longest PCGEM1 cDNA clone, SEQ ID NO:1 (Figure 8), revealed 1643 nucleotides with a potential polyadenylation site, ATTAAA, close to the 3' end followed by a poly (A) tail. As noted above, although initial searching of PCGEM1 gene in publically available DNA databases (e.g., National Center for Biotechnology Information) using the BLAST program did not reveal any homology, a recent search of the HTGS database revealed perfect homology of PCGEM1 (using cDNA of SEQ ID NO: 1) to a chromosome 2 derived uncharacterized, unfinished genomic sequence (accession # AC 013401). One of the cDNA clones, SEQ ID NO:2 (Figure 9), contained a 123 bp insertion at 278, and this inserted sequence showed strong homology (87%) to Alu sequence. It is likely that this clone represented the premature transcripts. Sequencing of several clones from RT-PCR further confirmed the presence of the two forms of transcripts.

Sequence analysis did not reveal any significant long open reading frame in both strands. The longest ORF in the sense strand was 105 nucleotides (572-679) encoding 35 amino acid peptides. However, the ATG was not in a strong context of initiation. Although we could not rule out the coding capacity for a very small peptide, it is possible that PCGEM1 may function as a non-coding RNA.

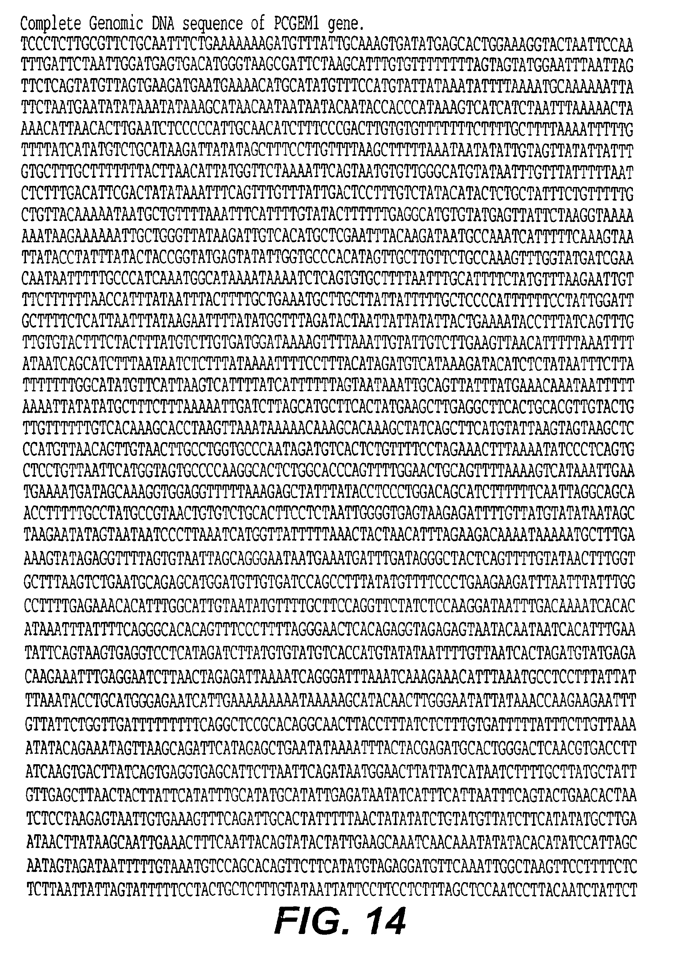

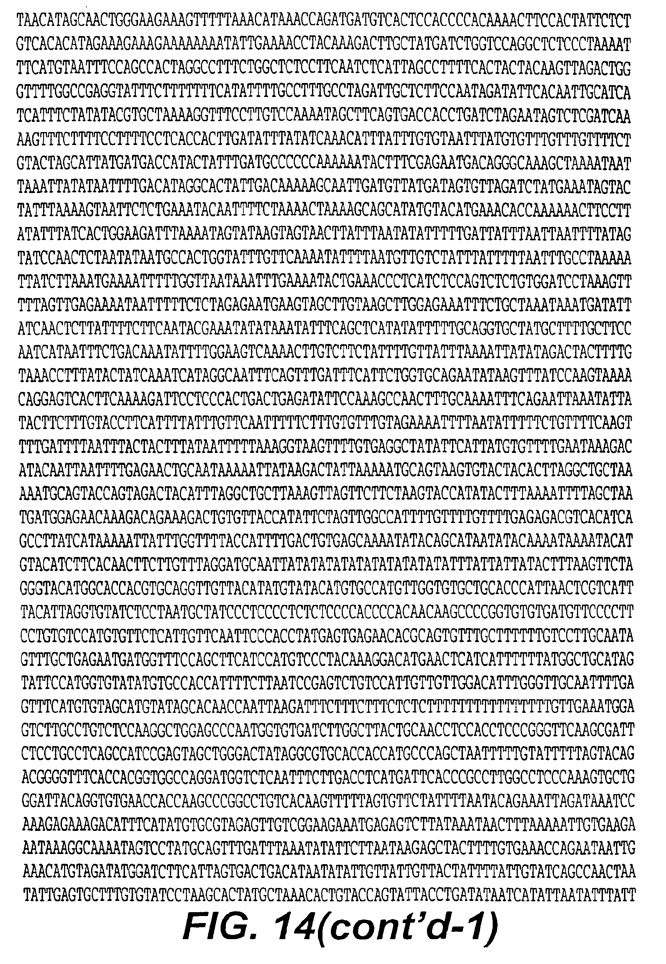

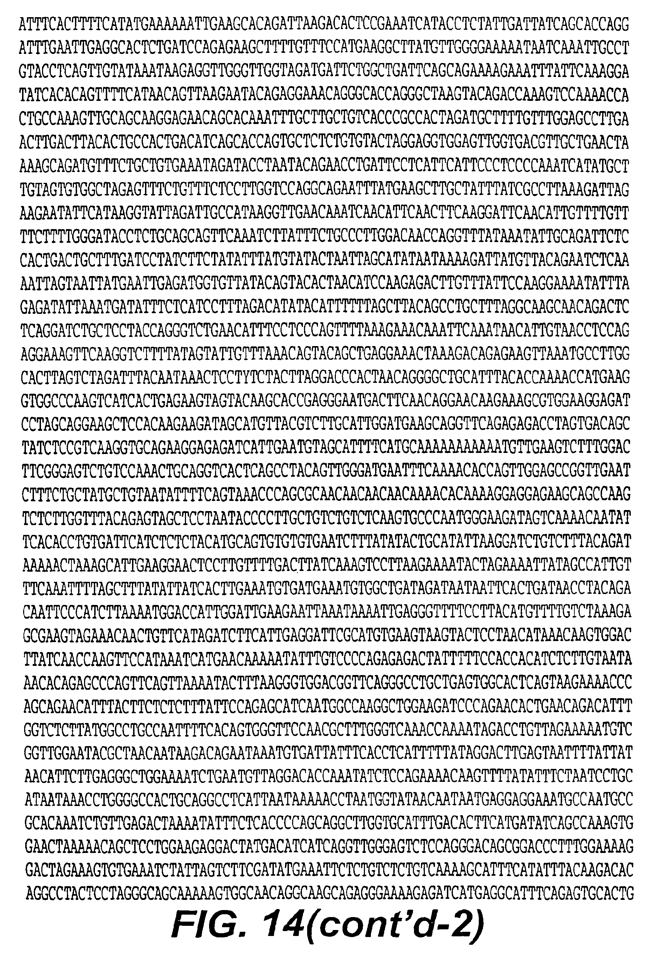

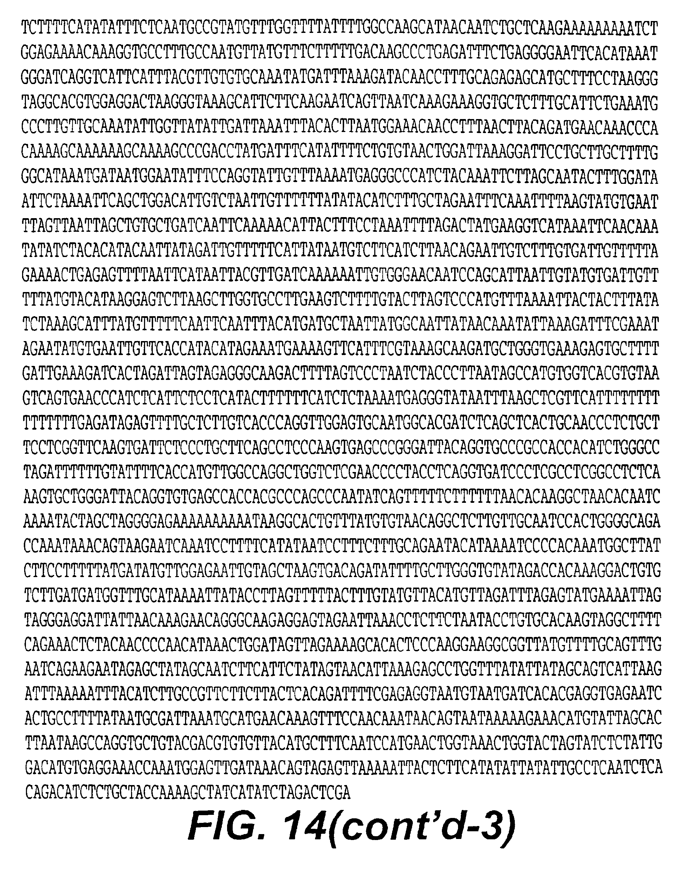

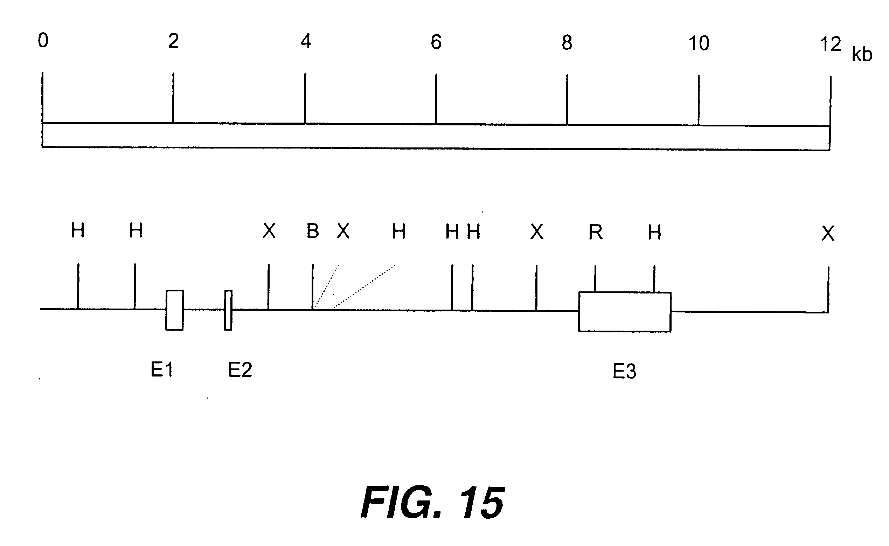

The sequence of PCGEM1 DNA has been verified by several approaches including characterization of several clones of PCGEM1 and analysis of PCGEM1 cDNAs amplified from normal prostate tissue and prostate cancer cell lines. We have also obtained the genomic clones of PCGEM1, which has helped to confirm the PCGEM1 cDNA sequence. The complete genomic DNA sequence of PCGEM1 (SEQ ID NO:8) is shown in Figure 14. In Figure 14 (and in the accompanying Sequence Listing), "Y" represents any one of the four nucleotide bases, cylosine, thymine, adenine, or guanine. Comparison of the cDNA and genomic sequences revealed the organization of the PCGEM1 transcription unit from three exons (Figure 15: E, Exon; B: BamHI; H: HindIII; X: XbaI; R: EcoRI).

### EXAMPLE 3: Mapping the Location of PCGEM1

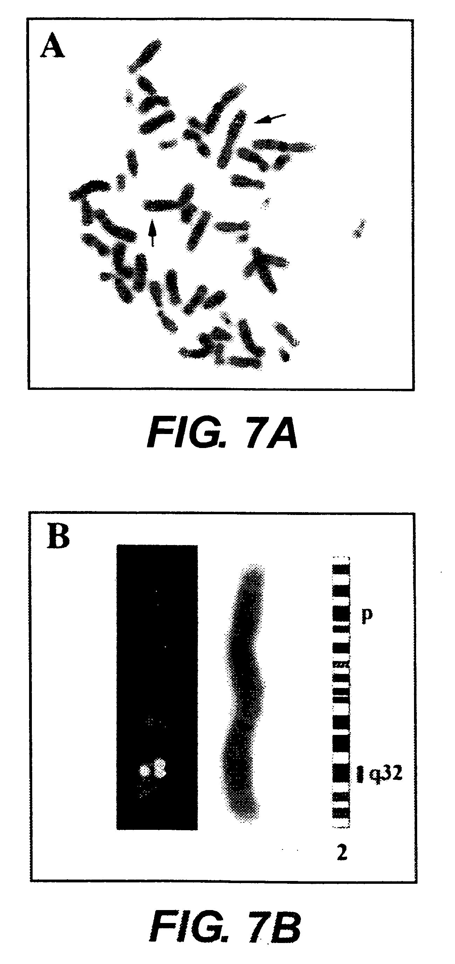

Using fluorescent *in situ* hybridization and the PCGEM1 genomic DNA as a probe, we mapped the location of PCGEM1 on chromosome 2q to specific region 2q32 (Figure 7A). Specifically, a Bacterial Artificial Chromosome (BAC) clone containing the PCGEM1 genomic sequence was isolated by custom services of Genome Systems (St. Louis, Mo). PCGEM1-Bac clone 1 DNA was nick translated using spectrum orange (Vysis) as a direct label and flourescent *in situ* hybridization was done using this probe on normal human male metaphase chromosome spreads. Counterstaining was done and chromosomal localization was determined based on the G-band analysis of inverted 4',6-diamidino-2-phenylindole (DAPI) images. (Figure 7B: a DAPI counter-stained chromosome 2 is shown on the left; an inverted DAPI stained chromosome 2 shown as G-bands is shown in the center; an ideogram of chromosome 2 showing the localization of the signal to band 2q32(bar) is shown on the right.) NU200 image acquisition and registration software was used to create the digital images. More than 20 metaphases were analyzed.

### EXAMPLE 4: Analysis of PCGEM1 Gene Expression in Prostate Cancer

To further characterize the tumor specific expression of the PCGEM1 fragment, and also to rule out individual variations of gene expression alterations commonly observed in tumors, the expression of the PCGEM1 fragment was evaluated on a test panel of matched tumor and normal RNAs derived from the microdissected tissues of twenty prostate cancer patients.

Using the PCGEM1 cDNA sequence (SEQ ID NO:1), specific PCR primers (Sense primer 1 (SEQ ID NO: 5): 5' TGCCTCAGCCTCCCAAGTAAC 3' and Antisense primer 2 (SEQ ID NO: 6): 5' GGCCAAAATAAAACCAAACAT 3') were designed for RT-PCR assays. Radical prostatectomy derived OCT compound (Miles Inc. Elkhart, IN) embedded fresh frozen normal and tumor tissues from prostate cancer patients were characterized for histopathology by examining hematoxylin and eosin stained sections (46). Tumor and normal prostate tissues regions representing approximately equal number of epithelial cells were dissected out of frozen sections. DNA-free RNA was prepared from these tissues and used in RT-PCR analysis to detect PCGEM1 expression. One hundred nanograms of total RNA was reverse transcribed into cDNA using RT-PCR kit (Perkin-Elmer, Foster, CA). The PCR was performed using Amplitaq Gold from Perkin-Elmer (Foster, CA). PCR cycles used were: 95°C for 10 minutes, 1 cycle; 95°C for 30 seconds, 55°C for 30 seconds, 72°C for 30 seconds, 42 cycles, and 72°C for 5 minutes, 1 cycle followed by a 4°C storage. Epithelial cell-associated cytokeratin 18 was used as an internal control.

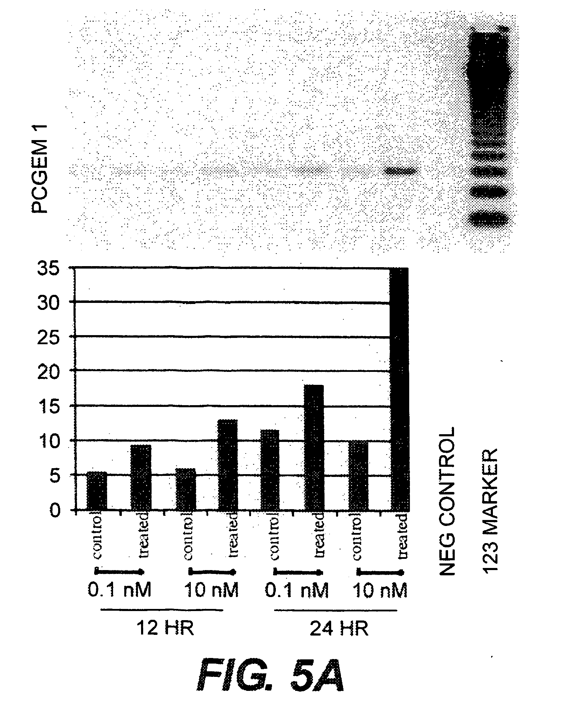

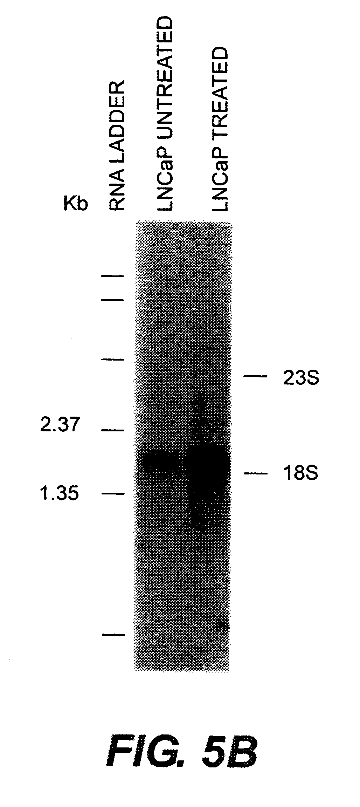

RT-PCR analysis of microdissected matched normal and tumor tissue derived RNAs from 23 CaP patients revealed tumor associated overexpression of PCGEM1 in 13 (56%) of the patients (Figure 5). Six of twenty-three (26%) patients did not exhibit detectable PCGEM1 expression in either normal or tumor tissue derived RNAs. Three of twenty-three (13%) tumor specimens showed reduced expression in tumors. One of the patients did not exhibit any change. Expression of housekeeping genes, cytokeratin-18 (Figure 3) and glyceraldehyde-3-phosphate dehydrogenase (GAPDH) (data not shown) remained constant in tumor and normal specimens of all the patients (Figure 3). These results were further confirmed by another set of PCGEM1 specific primers (Sense Primer 3 (SEQ ID NO: 7): 5' TGGCAACAGGCAAGCAGAG 3' and Antisense Primer 2 (SEQ ID NO: 6): 5' GGCCAAAATAAAACCAAACAT 3'). Four of 16 (25%) patients did not exhibit detectable PCGEM1 expression in either normal or tumor tissue derived RNAs. Two of 16 (12.5%) tumor specimens showed reduced expression in tumors. These results of PCGEM1 expression in tumor tissues could be explained by the expected individual variations between tumors of different patients. Most importantly, initial DD observations were confirmed by showing that 45% of patients analyzed did exhibit over expression of PCGEM1 in tumor prostate tissues when compared to corresponding normal prostate tissue of the same individual.

### EXAMPLE 5: In situ Hybridization

*In situ* hybridization was performed essentially as described by Wilkinson and Green (48). Briefly, OCT embedded tissue slides stored at -80°C were fixed in 4% PFA (paraformaldehyde), digested with proteinase K and then again fixed in 4% PFA. After washing in PBS, sections were treated with 0.25% acetic anhydride in 0.1M triethanolamine, washed again in PBS, and dehydrated in a graded ethanol series. Sections were hybridized with ³⁵S-labeled riboprobes at 52°C overnight. After washing and RNase A treatment, sections were dehydrated, dipped into NTB-2 emulsion and exposed for 11 days at 4°C. After development, slides were lightly stained with hematoxylin and mounted for microscopy. In each section, PCGEM1 expression was scored as percentage of cells showing ³⁵S signal: 1+, 1-25%; 2+, 25-50%; 3+, 50-75%, 4+, 75-100%.

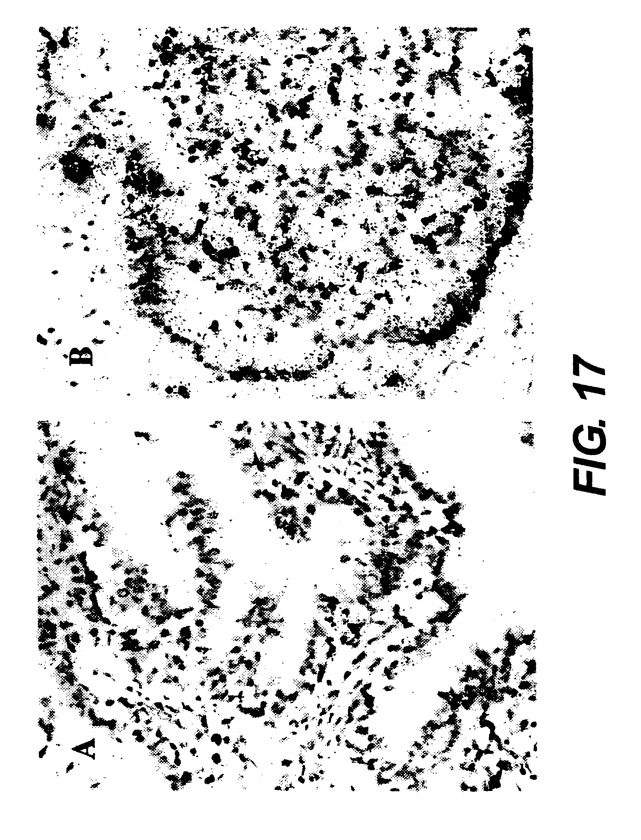

Paired normal (benign) and tumor specimens from 13 patients were tested using *in situ* hybridization. A representative example is shown in Figure 17. In 11 cases (84%) tumor associated elevation of PCGEM1 expression was detected. In 5 of these 11 patients the expression of PCGEM1 increased to 1+ in the tumor area from an essentially undetectable level in the normal area (on the 0 to 4+ scale). Tumor specimens from 4 of 11 patients scored between 2+ (example shown in Figure 17B) and 4+. Two of 11 patients showed focal signals with 3+ score in the tumor area, and one of these patients had similar focal signal (2+) in an area pathologically designated as benign. In the remaining 2 of the 13 cases there was no detectable signal in any of the tissue areas tested. The results indicate that PCGEM1 expression appears to be restricted to glandular epithelial cells. (Figure 17 shows an example of *in situ* hybridization of ³⁵S labeled PCGEM1 riboprobe to matched normal (A) versus tumor (B) sections of prostate cancer patients. The light gray areas are hematoxylin stained cell bodies, the black dots represent the PCGEM1 expression signal. The signal is background level in the normal (A), 2+ level in the tumor (B) section. The magnification is 40x.)

### EXAMPLE 6: PCGEM1 Gene Expression in Prostate Tumor Cell Lines

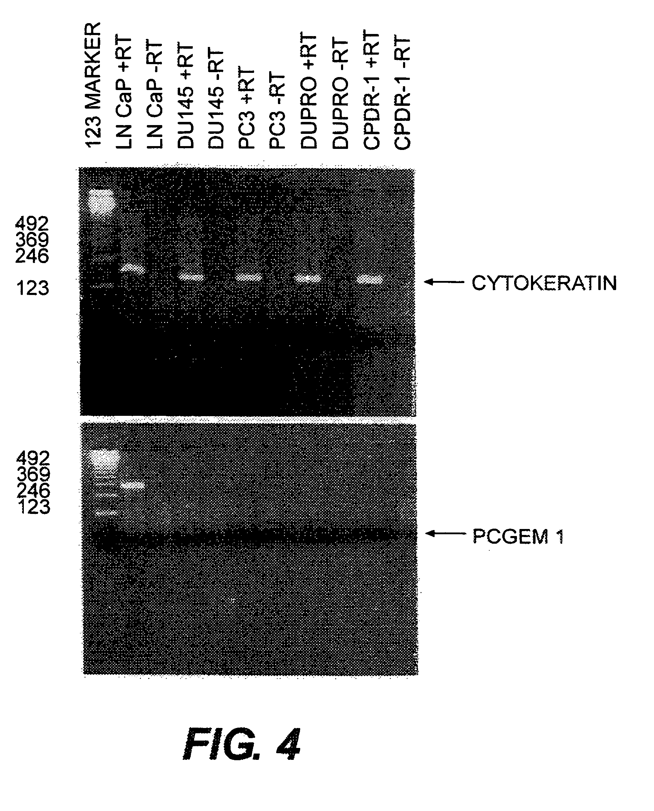

PCGEM1 gene expression was also evaluated in established prostate cancer cell lines: LNCaP, DU145, PC3 (all from ATCC), DuPro (available from Dr. David Paulson, Duke University, Durham, NC), and an E6/E7 - immortalized primary prostate cancer cell line, CPDR1 (47). CPDR1 is a primary CaP derived cell line immortalized by retroviral vector, LXSN 16 E6 E7, expressing E6 and E7 gene of the human papilloma virus 16. LNCaP is a well studied, androgen-responsive prostate cancer cell line, whereas DU145, PC3, DuPro and CPDR1 are androgen-independent and lack detectable expression of the androgen receptor. Utilizing the RT-PCR assay described above, PCGEM1 expression was easily detectable in LNCaP (Figure 4). However, PCGEM1 expression was not detected in prostate cancer cell lines DU145, PC3, DuPro and CPDR. Thus, PCGEM1 was expressed in the androgen-responsive cell line but not in the androgen-independent cell lines. These results indicate that hormones, particularly androgen, may play a key role in regulating PCGEM1 expression in prostate cancer cells. In addition, the results suggest that PCGEM1 expression may be used to distinguish between hormone responsive tumor cells and more aggressive hormone refractory tumor cells.

To test if PCGEM1 expression is regulated by androgens, we performed experiments evaluating PCGEM1 expression in LNCaP cells (ATCC) cultured with and without androgens. Total RNA from LNCaP cells, treated with synthetic androgen R1881 obtained from (DUPONT, Boston, MA), were analyzed for PCGEM1 expression. Both RT-PCR analysis (Figure 5a) and Northern blot analysis (Figure 5b) were conducted as follows.

LNCaP cells were maintained in RPMI 1640 (Life Technologies, Inc., Gaithersburg, MD) supplemented with 10% fetal bovine serum (FBS, Life Technologies, Inc., Gaithersburg, MD) and experiments were performed on cells between passages 20 and 35. For the studies of NKX3.1 gene expression regulation, charcoal/dextran stripped androgen-free FBS (cFBS, Gemini Bio-Products, Inc., Calabasas, CA) was used. LNCaP cells were cultured first in RPMI 1640 with 10% cFBS for 4 days and then stimulated with a non-metabolizable androgen analog R1881 (DUPONT, Boston, MA) at different concentrations for different times as shown in Figure 5A. LNCaP cells identically treated but without R1881 served as control. Poly A+ RNA derived from cells treated with/without R1881 was extracted at indicated time points with RNAzol B (Tel-Test, Inc, TX) and fractionated (2µg/lane) by running on 1% formaldehyde-agarose gel and transferred to nylon membrane. Northern blots were analyzed for the expression of PCGEM1 using the nucleic acid molecule set forth in SEQ ID NO: 4 as a probe. The RNA from LNCaP cells treated with R1881 and RNA from control LNCaP cells were also analyzed by RT-PCR assays as described in Example 4.

As set forth in Figures 5a and 5b, PCGEM1 expression increases in response to androgen treatment. This finding further supports the hypothesis that the PCGEM1 expression is regulated by androgens in prostate cancer cells.

### EXAMPLE 7: Tissue Specificity of PCGEM1 Expression

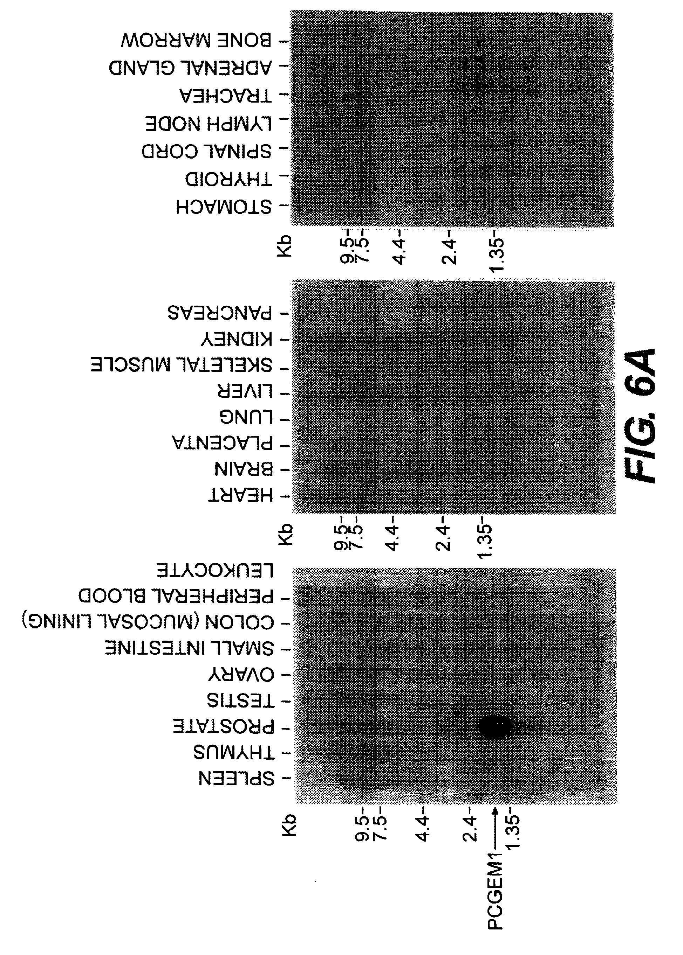

Multiple tissue Northern blots (Clontech, CA) conducted according to the manufacturer's directions revealed prostate tissue-specific expression of PCGEM1. Polyadenylate RNAs of 23 different human tissues (heart, brain, placenta, lung, liver skeletal muscle, kidney, pancreas, spleen, thymus, prostate, testis, ovary, small intestine, colon, peripheral blood, stomach, thyroid, spinal cord, lymph node, trachea, adrenal gland and bone marrow) were probed with the 530 base pair PCGEM1 DNA fragment (nucleotides 410 to 940 of SEQ ID NO:1). A 1.7 kilobase mRNA transcript hybridized to the PCGEM1 probe in prostate tissue (Figure 6a). Hybridization was not observed in any of the other human tissues (Figure 6a). Two independent experiments revealed identical results.

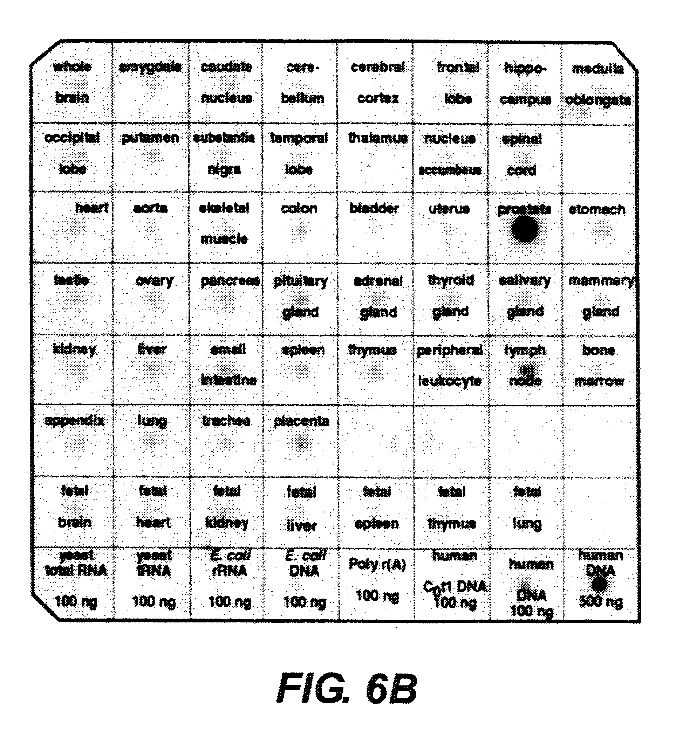

Additional Northern blot analyses on an RNA master blot (Clontech, CA) conducted according to the manufacturer's directions confirm the prostate tissue specificity of the PCGEM1 gene (Figure 6b). Northern blot analyses reveal that the prostate tissue specificity of PCGEM1 is comparable to the well known prostate marker PSA (77mer oligo probe) and far better than two other prostate specific genes PSMA (234 bp fragment from PCR product) and NKX3.1 (210 bp cDNA). For instance, PSMA is expressed in the brain (37) and in the duodenal mucosa and a subset of proximal renal tubules (38). While NKX3.1 exhibits high levels of expression in adult prostate, it is also expressed in lower levels in testis tissue and several other tissues (39).

### EXAMPLE 8: Biologic functions of the PCGEM1

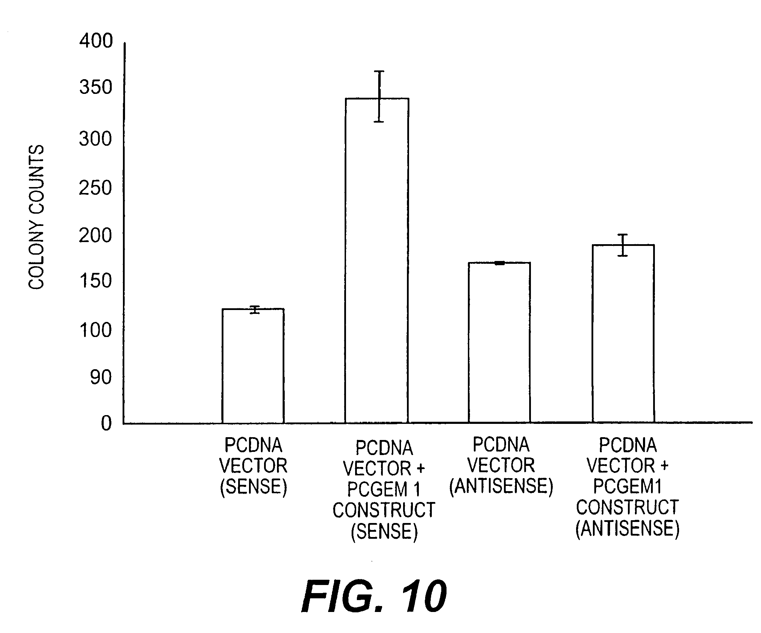

The tumor associated PCGEM1 overexpression suggested that the increased expression of PCGEM1 may favor tumor cell proliferation. NIH3T3 cells have been extensively used to define cell growth promoting functions associated with a wide variety of genes (40-44). Utilizing pcDNA3.1/Hygro(+/-)(Invitrogen, CA), PCGEM1 expression vectors were constructed in sense and anti-sense orientations and were transfected into NIH3T3 cells, and hygromycin resistant colonies were counted 2-3 weeks later. Cells transfected with PCGEM1 sense construct formed about 2 times more colonies than vector alone in three independent experiments (Figure 10). The size of the colonies in PCGEM1 sense construct transfected cells were significantly larger. No appreciable difference was observed in the number of colonies between anti-sense PCGEM1 constructs and vector controls. These promising results document a cell growth promoting/cell survival function(s) associated with PCGEM1.

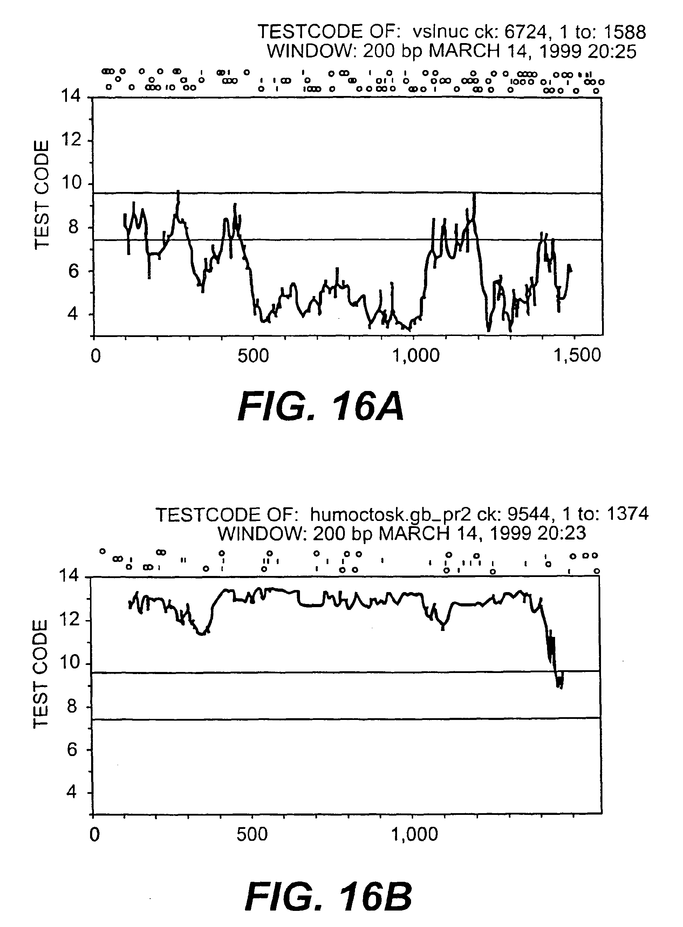

The function of PCGEM1, however, does not appear to be due to protein expression. To assess this hypothesis, we used the TestCode program (GCG Wisconsin Package, Madison, WI), which identifies potential protein coding sequences of longer than 200 bases by measuring the non-randomness of the composition at every third base, independently from the reading frames. Analysis of the PCGEM1 DNA sequence revealed that, at greater than 95% confidence level, the sequence does not contain any region with protein coding capacity (Figure 16A). Similar results were obtained when various published non-coding RNA sequences were analyzed with the TestCode program (data not shown), while known protein coding regions of similar size i.e., alpha actin (Figure 16B) can be detected with high fidelity. (In Figure 16, evaluation of the coding capacity of the PCGEM1 (A) and the human alpha actin (B), is performed independently from the reading frame, by using the TestCode program. The number of base pairs is indicated on the X- axis, the TestCode values are shown on the Y-axis. Regions of longer than 200 base pairs above the upper line (at 9.5 value) are considered coding, under the lower line (at 7.3 value) are considered non-coding, at a confidence level greater than 95%.)

The Codon Preference program (GCG Wisconsin Package, Madison, WI), which locates protein coding regions in a reading frame specific manner further suggested the absence of protein coding capacity in the PCGEM1 gene (see www.cpdr.org). *In vitro* transcription/translation of PCGEM1 cDNA did not produce a detectable protein/peptide. Although we can not unequivocally rule out the possibility that PCGEM1 codes for a short unstable peptide, at this time both experimental and computational approaches strongly suggest that PCGEM1 cDNA does not have protein coding capacity. (It should be recognized that conclusions regarding the role of PCGEM1 are speculative in nature, and should not be considered limiting in any way.

The most intriguing aspect of PCGEM1 characterization has been its apparent lack of protein coding capacity. Although we have not completely ruled out the possibility that PCGEM1 codes for a short unstable peptide, careful sequencing of PCGEM1 cDNA and genomic clones, computational analysis of PCGEM1 sequence, and *in vitro* transcription/translation experiments (data not shown) strongly suggest a non-coding nature of PCGEM1. It is interesting to note that an emerging group of novel mRNA-like non-coding RNAs are being discovered whose function and mechanisms of action remain poorly understood (49). Such RNA molecules have also been termed as "RNA riboregulators" because of their function(s) in development, differentiation, DNA damage, heat shock responses and tumorigenesis (40-42, 50). In the context of tumorigenesis, the *H19, His*-1 and *Bic* genes code for functional non-coding mRNAs (50). In addition, a recently reported prostate cancer associated gene, DD3 also appears to exhibit a tissue specific non-coding mRNA (51). In this regard it is important to point out that PCGEM1 and DD3 may represent a new class of prostate specific genes. The recent discovery of a steroid receptor co-activator as an mRNA, lacking protein coding capacity further emphasizes the role of RNA riboregulators in critical biochemical function(s) (52). Our preliminary results showed that PCGEM1 expression in NIH3T3 cells caused a significant increase in the size of colonies in a colony forming assay and suggests that PCGEM1 cDNA confers cell proliferation and/or cell survival function(s). Elevated expression of PCGEM1 is prostate cancer cells may represent a gain in function favoring tumor cell proliferation/survival. On the basis of our first characterization of PCGEM1 gene, we propose that PCGEM1 belongs to a novel class of prostate tissue specific genes with potential functions in prostate cell biology and the tumorigenesis of the prostate gland.

In summary, utilizing surgical specimens and rapid differential display technology, we have identified candidate genes of interest with differential expression profile in prostate cancer specimens. In particular, we have identified a novel nucleotide sequence, PCGEM1, with no match in the publicly available DNA databases (except for the homology shown in the high throughput genome sequence database, discussed above). A PCGEM1 cDNA fragment detected a 1.7 kb mRNA on Northern blots with selective expression in prostate tissue. Furthermore, this gene was found to be up-regulated by the synthetic androgen, R1881. Careful analysis of microdissected matched tumor and normal tissues further revealed PCGEM1 over-expression in a significant percentage of prostate cancer specimens. Thus, we have provided a gene with broad implications for the diagnosis, prevention, and treatment of prostate cancer.

### REFERENCES

1. Parker SL, Tong T, Bolden S, and Wingo PA: Cancer statistics. CA Cancer J. Clin., 46:5-27, 1996.
2. Visakorpi T, Kallioniemi OP, Koivula T and Isola J: New prognostic factors in prostate carcinoma. Eur. Uro., 24:438-449, 1993.
3. Mostofi FK: Grading of prostate carcinoma. Cancer Chemothera Rep., 59:111, 1975.
4. Lu-Yao GL, McLerran D, Wasson J, Wennberg JE: An assessment of radical prostatectomy. Time trends, Geographical Variations and Outcomes. JAMA, 269:2633-2636, 1993.
5. Partin AW and Oesterling JE: The clinical usefulness of prostate-specific antigen: update 1994, J. Urol., 152:1358-1368, 1994.
6. Wasson JH, Cushman CC, Bruskewitz RC, Littenberg B, Mulley AG, and Wennberg JE: A structured literature review of treatment for localized CaP. Arch. Fam. Med., 2:487-493, 1993.
7. Weinberg RA: How cancer arises. Sci. Amer., 9, 62-70, 1996.
8. Bostwick DG: High grade prostatic intraepithelial neoplasia: The most likely precursor of prostate cancer. Cancer, 75:1823-1836, 1995.
9. Bostwick DG, Pacelli A, Lopez-Beltran A: Molecular Biology of Prostatic Intraepithelial Neoplasia. The Prostate, 29:117-134, 1996.
10. Pannek J, Partin AW: Prostate specific antigen: What's new in 1997. Oncology, 11:1273-1278, 1997.
11. Partin AW, Kattan MW, Subong EN, Walsh PC, Wojno KJ, Oesterling JE, Scardino PT, Pearson JD: Combination of prostate specific antigen, clinical stage, and Gleason score to predict pathological stage of localized prostate cancer. A multi-institutional update. JAMA, 277:1445-1451, 1997.
12. Gomella LG, Raj GV, Moreno JG: Reverse transcriptase polymerase chain reaction for prostate specific antigen in management of prostate cancer. J Urol., 158:326-337, 1997.
13. Gao CL, Dean RC, Pinto A, Mooneyhan R, Connelly RR, McLeod DG, Srivastava, S, Moul JW: Detection of PSA-expressing prostatic cells in bone marrow of radical prostatectomy patients by sensitive reverse transcriptase-polymerase chain reaction (RT-PCR). 1998 International Symposium on Biology of Prostate growth, National Institutes of Health, p. 83, 1998.
14. Garnick MB, Fair WR: Prostate cancer. Sci. Amer., 75-83, 1998.
15. Moul JW, Gaddipati J, and Srivastava S: 1994. Molecular biology of CaP. Oncogenes and tumor suppressor genes. Current Clinical Oncology: CaP. (Eds. Dawson, N.A. and Vogelzang, N.J.), Wiley-Liss Publications, 19-46.
16. Lalani E-N, Laniado ME and Abel PD: Molecular and cellular biology of prostate cancer. Cancer and Mets. Rev. 16:29-66, 1997.
17. Shi XB, Gumerlock PH, deVere White RW: Molecular Biology of CaP World J. Urol; 14, 318-328, 1996.
18. Heidenberg HB, Bauer JJ, McLeod DG, Moul JW and Srivastava S: The role of p53 tumor suppressor gene in CaP: a possible biomarker? Urology, 48:971-979, 1996.
19. Bova GS and Issacs WB: Review of allelic loss and gain in prostate cancer. World J Urol., 14:338-346, 1996.
20. Issacs WB and Bova GS: Prostate Cancer: The Genetic Basis of Human Cancer. Eds. Vogelstein B, and Kinzler KW, McGraw-Hill Companies, Inc., pp. 653-660, 1998.
21. Srivastava S and Moul JW: Molecular Progression of Prostate Cancer. Advances in Oncobiology. (In Press) 1998.
22. Sakr WA, Macoska JA, Benson P, Benson DJ, Wolman SR, Pontes JE, and Crissman: Allelic loss in locally metastatic, multi-sampled prostate cancer. Cancer Res., 54:3273-3277, 1994.
23. Mirchandani D, Zheng J, Miller GL, Ghosh AK, Shibata DK, Cote RJ and Roy-Burman P: Heterogeneity in intratumor distribution of p53 mutations in human prostate cancer. Am. J. Path. 147:92-101, 1995.
24. Bauer JJ, Moul JW, and McLeod DG: CaP: Diagnosis, treatment, and experience at one tertiary medical center, 1989-1994. Military Medicine, 161:646-653,1996.
25. Bauer JJ, Connelly RR, Sesterhenn IA, Bettencourt MC, McLeod DG, Srivastava S, Moul JW: Biostatistical modeling using traditional variables and genetic biomarkers predicting the risk of prostate cancer recurrence after radical prostatectomy. Cancer, 79:952-962, 1997.
26. Bauer JJ, Connelly RR, Sesterhenn IA, DeAusen JD, McLeod DG, Srivastava S, Moul JW: Biostatistical modeling using traditional preoperative and pathological prognostic variables in the selection of men at high risk of disease recurrence after radical prostatectomy. J. Urol., 159(3):929-933, 1998
27. Sager R: Expression genetics in cancer: Shifting the focus from DNA to RNA. Proc Natl. Acad Sci. USA, 94:952-957, 1997
28. Strausberg RL, Dahl CA, and Klausner RD: New opportunities for uncovering the molecular basis of cancer. Nature Genetics, 15:415-16, 1997.
29. Liang, Peng, and Pardec AB: Differential display of eukaryotic messenger RNA by means of the polymerase chain reaction. Science 257:967-971, 1992.
30. Velculescu VE, Zhang L, Vogelstein B, and Kinzler KW: Serial analysis of gene expression Science, 270:484-487, 1995.
31. Chena M, Shalon DS, Davis RW, and Brown PO: Quantitative monitoring of gene expression patterns with a complementary DNA microarrays. Science, 270:467-470, 1995.
32. Liu AY, Corey E, Vessella RL, Lange PH, True LD, Huang GM, Nelson PS and Hood L: Identification of differentially expressed prostate genes: Increased expression of transcription factor ETS-2 in prostate cancer. The Prostate 30:145-153, 1997.
33. Chuaqui RF, Englert CR, Strup SE, Vocke CD, Zhuang Z, Duray PH, Bostwick DG, Linehan WM, Liotta LA and Emmert-Buck MR: Identification of a novel transcript up-regulated in a clinically aggressive prostate carcinoma. Urology, 50:302-307, 1997.
34. Thigpen AE, Cala KM, Guileyardo JM, Molberg KH, McConnell JD, and Russell DW: Increased expression of early growth response-1 messenger ribonucleic acid in prostate adenocarcinoma. J. Urol., 155:975-981, 1996.
35. Wang FL, Wang Y, Wong WK, Liu Y, Addivinola FJ, Liang P, Chen LB, Kantoff PW and Pardee AB: Two differentially expressed genes in normal human prostate tissues and in carcinoma. Cancer Res., 56:3634-3637, 1996.
36. Schleicher RL, Hunter SB, Zhang M, Zheng M, Tan W, Bandea CI, Fallon MT, Bostwick DG, and Varma VA: Neurofilament heavy chain-like messenger RNA and protein are present in benign prostate and down regulated in prostate carcinoma. Cancer Res., 57:3532-3536, 1997.
37. O'Keefe, DS, Su, SL, Bacich DJ, Horiguchi Y, Luo Y, Powell CT, Zandvliet D, Russell PJ, Molloy PL, Nowak, NJ, Shows, TB, Mullins, C, Vonder Haar RA, Fair WR, and Heston WD: Mapping, genomic organization and promoter analysis of the human prostate-specific membrane antigen gene. Biochim Biophys Acta, 1443(1-2):113-127, 1998.
38. Silver DA, Pellicer I, Fair WR, Heston, WD, and Cordon-Cardo C: Prostate-specific membrane antigen expression in normal and malignant human tissues. Clin Cancer Res, 3(1):81-85, 1997.
39. He WW, Sciavolino PJ, Wing J, Augustus M, Hudson P, Meissner PS, Curtis RT, Shell BK, Bostwick DG, Tindall DJ, Gelmann EP, Abate-Shen C, and Carter KC: A novel prostate-specific, androgen-regulated homeobox gene (NKX3.1) that maps to 8p21, a region frequently deleted in prostate cancer. Genomics 43(1):69-77, 1997.
40. Crespi MD, Jurkevitch E, Poiret M, d'Aubenton-Carafa Y, Petrovics G, Kondorosi E, and Kondorosi A: Enod 40, a gene expressed during nodule organogenesis, codes for a non-translatable RNA involved in plant growth. The EMBO J 13:5099-5112, 1994.
41. Velleca MA, Wallace MC and Merlie JP: A novel synapse-associated non-coding RNA. Mol. Cell Bio. 14:7095-7104, 1994.
42. Takeda K. Ichijoh, Fujii M, Mochida Y, Saitoh M, Nishitoh H, Sampath TK and Miyazonok: Identification of a novel bone morphogenetic protein responsive gene that may function as non-coding RNA. J. Biol. Chem. 273:17079-17085, 1998.
43. Van de Sande K, Pawlowski K, Czaja I, Wieneke U, et al: Modification of phytohormone response by a peptide encode by ENOD 40 of legumes and a non-legume. Science 273:370-373.
44. Hao Y, Crenshaw T, Moulton T, Newcomb E and Tycko B: Tumor suppressor activity of H19RNA. Nature. 365:764-767, 1993.
45. Neumaier M, Gerhard M, Wagener C: Diagnosis of micrometastases by the amplification of tissue specific genes. Gene. 159(1) :43-47, 1995.
46. Gaddipati J, McLeod D, Sesterhenn I, Hussussian C, Tong Y, Seth P, Dracopoli N, Moul J and Srivastava S: Mutations of the p16 gene product are rare in prostate cancer. The Prostate. 30:188-194, 1997.
47. Davis LD, Sesterhenn IA, Moul JW and Srivastava S: Characterization of prostate cancer cells immortalized with E6/E7 genes. Int. Symp. On Biol. Of Prost. Growth Proceedings, National Institutes of Health., 77, 1998.
48. Wilkinson, D., & Green, J. (1990) in Post implantation Mammalian Embryos, eds. Copp, A.J. & Cokroft, D.L. (Oxford University Press, London), pp. 155-171.
49. Erdmann, V.A., Szymanski, M., Hochberg, A., de Groot, N., & Barciszewski, J. (1999) Nucleic Acids Research 27, 192-195.
50. Askew, D.S., & Xu, F. (1999) Histol Histopatho. 14,235-241.
51. Bussemakers, M.J.H., Van Bokhoven, A., Verhaegh, G.W., Smit, F.P., Karthaus, H.F., Schalken, J.A., Debruyne, F.M., Ru, N., & Isaacs, W.B. (1999) Cancer Res. 59, 5975-5979.
52. Lanz, R.B., McKenna, N.J., Onate, S.A., Albrecht, U., Wong, J., Tsai, S.Y., Tsai, M.J., & O'Mally, B.W. (1999) Cell 97, 17-27.
53. Srikantan V, Zou Z, Petrovics G, Xu L, Augustus M, Davis L, Livezey JR, Connell T, Sesterhenn IA, Yoshino K, Buzard GS, Mostofi FK, McLeod DG, Moul JW, and Srivastava S: PCGEM 1: A Novel Prostate Specific Gene is Overexpressed in Prostate Cancer. Submitted to Proceedings of the National Academy of Sciences*.*

## Claims

1. An isolated nucleic acid molecule selected from the group consisting of:
(a) the polynucleotide sequence consisting of SEQ ID NO:1 or SEQ ID NO:2;
(b) the complement of (a); and
(c) a nucleic acid sequence consisting of SEQ ID NO: 4, SEQ ID NO: 6, SEQ ID NO: 7, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22.

2. A recombinant vector comprising a nucleic acid molecule according to Claim 1, wherein the vector directs the expression of said nucleic acid molecule.

3. A host cell comprising a vector according to Claim 2, wherein said host cell is selected from bacterial cells, yeast cells and animal cells, provided that when said animal cells are human cells, they are isolated.

4. An *in vitro* method of detecting prostate cancer in a patient, the method comprising:
(a) detecting a mRNA sequence in a biological sample from the patient, by hybridising the mRNA sequence to SEQ ID NO:1 or SEQ ID NO:2 under conditions of high stringency; and
(b) correlating the amount of the mRNA sequence in the sample with the presence of prostate cancer in the patient.

5. An *in vitro* method of detecting prostate cancer in a patient, the method comprising:
(a) isolating a mRNA sequence from a sample from the patient;
(b) amplifying a cDNA comprising SEQ ID NO: 1 or a fragment thereof from the mRNA isolated in (a);
(c) incubating the amplified cDNA with a nucleic acid probe that hybridises to SEQ ID NO: 1 to form a duplex molecule that is both stable and selective;
(d) detecting hybridisation between the amplified cDNA and the probe; and
(e) correlating the amount of the mRNA sequence in the sample with the presence of prostate cancer in the patient.

6. A method according to Claim 5, wherein the cDNA is amplified with at least two nucleotide sequences selected from: SEQ ID NO: 5, SEQ ID NO:6; SEQ ID NO:7, SEQ ID NO:9, SEQ ID NO:10, SEQ ID NO:11, SEQ ID NO:12; SEQ ID NO:13, SEQ ID NO:14, SEQ ID NO:15, SEQ ID NO:16, SEQ ID NO:17; SEQ ID NO:18, SEQ ID NO:19, SEQ ID NO:20, SEQ ID NO:21 and SEQ ID NO: 22.

7. A method according to Claim 6, wherein the at least two nucleotide sequences are SEQ ID NO: 15 and SEQ ID NO: 22.

8. A method according to Claim 4, wherein the biological sample is selected from blood, urine and prostate tissue.

9. A method according to Claim 8, wherein the biological sample is blood.

10. An *in vitro* method of measuring the responsiveness of prostate tissue to hormone-ablation therapy, wherein said method comprises:
(a) detecting a mRNA sequence in a biological sample from the patient, by hybridising the mRNA sequence to SEQ ID NO:1 or SEQ ID NO:2 under conditions of high stringency; and
(b) correlating the amount of the mRNA sequence in the sample with the presence of prostate cancer in the patient.

11. An isolated nucleic acid molecule consisting of a fragment of SEQ ID NO: 1,
wherein the fragment comprises at least 20 contiguous nucleotides of SEQ ID NO: 1.

12. The isolated nucleic acid molecule according to Claim 11, wherein the nucleic acid comprises at least 30 contiguous nucleotides of SEQ ID NO: 1.

13. The isolated nucleic acid molecule according to Claim 11, wherein the nucleic acid comprises at least 60 contiguous nucleotides of SEQ ID NO: 1.

14. An *in vitro* method of identifying an androgen-responsive cell line, the method comprising:
(a) incubating a cell line suspected of being androgen responsive with an androgen; and
(b) detecting a mRNA sequence in the cell line, by hybridising the mRNA sequence to SEQ ID NO:1 or SEQ ID NO:2 under conditions of high stringency;
wherein an increase in the mRNA sequence, as compared to an untreated cell line, correlates with the cell line being androgen responsive.

15. An *in vitro* method of identifying an androgen-responsive cell line, the method comprising:
(a) incubating a cell line suspected of being androgen responsive with an androgen;
(b) isolating a mRNA sequence from said cell line;
(c) amplifying a cDNA comprising SEQ ID NO: 1 or SEQ ID NO: 2 or a fragment thereof from the mRNA isolated in (b);
(d) incubating the amplified cDNA with a nucleic acid probe that hybridises to SEQ ID NO: 1 or SEQ ID NO: 2 to form a duplex molecule that is both stable and selective; and
(e) detecting hybridisation between the amplified cDNA and the probe;
wherein an increase in the mRNA sequence, as compared to an untreated cell line, correlates with the cell line being androgen responsive.

16. An *in vitro* method of measuring the responsiveness of a prostate tissue to hormone-ablation therapy, the method comprising:
(a) isolating mRNA from prostate tissue following hormone ablation therapy;
(b) amplifying a cDNA comprising SEQ ID NO: 1 or SEQ ID NO: 2 or a fragment thereof from the mRNA isolated in (a);
(c) incubating the amplified cDNA with a nucleic acid probe that hybridises to SEQ ID NO: 1 to form a duplex molecule that is both stable and selective; and
(d) detecting hybridisation between the amplified cDNA and the probe;
wherein a decrease in the mRNA sequence, as compared to untreated prostate tissue, correlates with the prostate tissue responding to hormone ablation therapy.

## Patentansprüche

1. Isoliertes Nukleinsäuremolekül ausgewählt aus der Gruppe bestehend aus:
(a) der Polynukleotidsequenz bestehend aus SEQ ID NO: 1 oder SEQ ID NO: 2;
(b) der Komplementärsequenz von (a); und
(c) einer Nukleinsäuresequenz bestehend aus SEQ ID NO: 4, SEQ ID NO: 6, SEQ ID NO: 7, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 oder SEQ ID NO: 22.

2. Rekombinanter Vektor umfassend ein Nukleinsäuremolekül nach Anspruch 1,
wobei der Vektor die Expression des Nukleinsäuremoleküls regelt.

3. Wirtszelle umfassend einen Vektor nach Anspruch 2, wobei die Wirtszelle ausgewählt ist aus Bakterienzellen, Hefezellen und Tierzellen, mit der Einschränkung, dass die Zellen isoliert sind, wenn die Tierzellen Humanzellen sind.

4. *In* vitro-Verfahren zum Nachweis von Prostatakrebs bei einem Patienten, wobei das Verfahren umfasst:
(a) Nachweisen einer mRNA-Sequenz in einer biologischen Probe von dem Patienten durch Hybridisieren der mRNA-Sequenz an SEQ ID NO: 1 oder SEQ ID NO: 2 unter Bedingungen hoher Stringenz; und
(b) Korrelieren der Menge der mRNA-Sequenz in der Probe mit dem Vorliegen von Prostatakrebs bei dem Patienten.

5. *In* vitro-Verfahren zum Nachweis von Prostatakrebs bei einem Patienten, wobei das Verfahren umfasst:
(a) Isolieren einer mRNA-Sequenz aus einer Probe von dem Patienten;
(b) Amplifizieren einer cDNA, die SEQ ID NO: 1 oder ein Fragment davon umfasst, aus der in (a) isolierten mRNA;
(c) Inkubieren der amplifizierten cDNA mit einer Nukleinsäuresonde, die an SEQ ID NO: 1 hybridisiert, zur Bildung eines doppelsträngigen Moleküls, das sowohl stabil als auch selektiv ist;
(d) Nachweisen von Hybridisierung zwischen der amplifizierten cDNA und der Sonde; und
(e) Korrelieren der Menge der mRNA-Sequenz in der Probe mit dem Vorliegen von Prostatakrebs bei dem Patienten.

6. Verfahren nach Anspruch 5, wobei die cDNA mit wenigstens zwei Nukleotidsequenzen amplifiziert wird, die ausgewählt sind aus: SEQ ID NO: 5, SEQ ID NO: 6, SEQ ID NO: 7, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 und SEQ ID NO: 22.

7. Verfahren nach Anspruch 6, wobei die wenigstens zwei Nukleotidsequenzen SEQ ID NO: 15 und SEQ ID NO: 22 sind.

8. Verfahren nach Anspruch 4, wobei die biologische Probe ausgewählt ist aus Blut, Urin und Prostatagewebe.

9. Verfahren nach Anspruch 8, wobei die biologische Probe Blut ist.

10. *In* vitro-Verfahren zur Messung des Ansprechens von Prostatagewebe auf Hormonablationstherapie, wobei das Verfahren umfasst:
(a) Nachweisen einer mRNA-Sequenz in einer biologischen Probe von dem Patienten durch Hybridisieren der mRNA-Sequenz an SEQ ID NO: 1 oder SEQ ID NO: 2 unter Bedingungen hoher Stringenz; und
(b) Korrelieren der Menge der mRNA-Sequenz in der Probe mit dem Vorliegen von Prostatakrebs bei dem Patienten.

11. Isoliertes Nukleinsäuremolekül bestehend aus einem SEQ ID NO: 1- Fragment,
wobei das Fragment wenigstens 20 aufeinanderfolgende Nukleotide von SEQ ID NO: 1 umfasst.

12. Isoliertes Nukleinsäuremolekül nach Anspruch 11, wobei die Nukleinsäure wenigstens 30 aufeinanderfolgende Nukleotide von SEQ ID NO: 1 umfasst.

13. Isoliertes Nukleinsäuremolekül nach Anspruch 11, wobei die Nukleinsäure wenigstens 60 aufeinanderfolgende Nukleotide von SEQ ID NO: 1 umfasst.

14. *In* vitro-Verfahren zum Identifizieren einer auf Androgene ansprechenden Zelllinie,
wobei das Verfahren umfasst:
(a) Inkubieren einer Zelllinie, von der vermutet wird, dass sie auf Androgene anspricht, mit einem Androgen; und
(b) Nachweisen einer mRNA-Sequenz in der Zelllinie durch Hybridisieren der mRNA-Sequenz an SEQ ID NO: 1 oder SEQ ID NO: 2 unter Bedingungen hoher Stringenz;
wobei eine Zunahme der mRNA-Sequenz im Vergleich mit einer unbehandelten Zelllinie damit korreliert, dass die Zelllinie auf Androgene anspricht.

15. *In* vitro-Verfahren zum Identifizieren einer auf Androgene ansprechenden Zelllinie,
wobei das Verfahren umfasst:
(a) Inkubieren einer Zelllinie, von der vermutet wird, dass sie auf Androgene anspricht, mit einem Androgen;
(b) Isolieren einer mRNA-Sequenz aus der Zelllinie;
(c) Amplifizieren einer cDNA, die SEQ ID NO: 1 oder SEQ ID NO: 2 oder ein Fragment davon umfasst, aus der in (b) isolierten mRNA;
(d) Inkubieren der amplifizierten cDNA mit einer Nukleinsäuresonde, die an SEQ ID NO: 1 oder SEQ ID NO: 2 hybridisiert, zur Bildung eines doppelsträngigen Moleküls, das sowohl stabil als auch selektiv ist; und
(e) Nachweisen von Hybridisierung zwischen der amplifizierten cDNA und der Sonde;
wobei eine Zunahme der mRNA-Sequenz im Vergleich mit einer unbehandelten Zelllinie damit korreliert, dass die Zelllinie auf Androgene anspricht.

16. *In* vitro-Verfahren zur Messung des Ansprechens von Prostatagewebe auf Hormonablationstherapie, wobei das Verfahren umfasst:
(a) Isolieren von mRNA aus Prostatagewebe nach Hormonablationstherapie;
(b) Amplifizieren einer cDNA, die SEQ ID NO: 1 oder SEQ ID NO: 2 oder ein Fragment davon umfasst, aus der in (a) isolierten mRNA;
(c) Inkubieren der amplifizierten cDNA mit einer Nukleinsäuresonde, die an SEQ ID NO: 1 hybridisiert, zur Bildung eines doppelsträngigen Moleküls, das sowohl stabil als auch selektiv ist; und
(d) Nachweisen von Hybridisierung zwischen der amplifizierten cDNA und der Sonde;
wobei eine Abnahme der mRNA-Sequenz im Vergleich mit unbehandeltem Prostatagewebe damit korreliert, dass das Prostatagewebe auf Hormonablationstherapie anspricht.

## Revendications

1. Molécule d'acide nucléique isolée choisie dans le groupe constitué :
(a) de la séquence polynucléotidique constituée de SEQ ID NO : 1 ou SEQ ID NO : 2 ;
(b) du complémentaire de (a) ; et
(c) d'une séquence d'acide nucléique constituée de SEQ ID NO : 4, SEQ ID NO : 6, SEQ ID NO : 7, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO: 17, SEQ ID NO : 18, SEQ ID NO: 19, SEQ ID NO : 20, SEQ ID NO : 21 ou SEQ ID NO : 22.

2. Vecteur recombinant comprenant une molécule d'acide nucléique selon la revendication 1, où le vecteur dirige l'expression de ladite molécule d'acide nucléique.

3. Cellule hôte comprenant un vecteur selon la revendication 2, où ladite cellule hôte est choisie parmi des cellules bactériennes, des cellules de levures et des cellules animales, à condition que lorsque lesdites cellules animales sont des cellules humaines, elles soient isolées.

4. Procédé *in vitro* de détection d'un cancer de la prostate chez un patient, le procédé comprenant :
(a) la détection d'une séquence d'ARNm dans un échantillon biologique provenant du patient, par hybridation de la séquence d'ARNm à SEQ ID NO : 1 ou SEQ ID NO : 2 dans des conditions de stringence élevée ; et
(b) la corrélation de la quantité de la séquence d'ARNm dans l'échantillon avec la présence d'un cancer de la prostate chez le patient.

5. Procédé *in vitro* de détection d'un cancer de la prostate chez un patient, le procédé comprenant :
(a) l'isolement d'une séquence d'ARNm à partir d'un échantillon provenant du patient ;
(b) l'amplification d'un ADNc comprenant SEQ ID NO : 1 ou un fragment de celle-ci à partir de l'ARNm isolé en (a) ;
(c) l'incubation de l'ADNc amplifié avec une sonde d'acide nucléique qui s'hybride à SEQ ID NO : 1 pour former une molécule duplex qui est à la fois stable et sélective ;
(d) la détection de l'hybridation entre l'ADNc amplifié et la sonde ; et
(e) la corrélation de la quantité de la séquence d'ARNm dans l'échantillon avec la présence d'un cancer de la prostate chez le patient.

6. Procédé selon la revendication 5, dans lequel l'ADNc est amplifié avec au moins deux séquences nucléotidiques choisies parmi SEQ ID NO : 5, SEQ ID NO : 6, SEQ ID NO : 7, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17 ; SEQ ID NO : 18, SEQ ID NO : 19, SEQ ID NO : 20, SEQ ID NO : 21 et SEQ ID NO : 22.

7. Procédé selon la revendication 6, dans lequel les aux moins deux séquences nucléotidiques sont SEQ ID NO : 15 et SEQ ID NO : 22.

8. Procédé selon la revendication 4, dans lequel l'échantillon biologique est choisi parmi le sang, l'urine et un tissu de la prostate.

9. Procédé selon la revendication 8, dans lequel l'échantillon biologique est du sang.

10. Procédé *in vitro* de mesure de la réactivité d'un tissu de la prostate à une hormonothérapie par ablation, où ledit procédé comprend :
(a) la détection d'une séquence d'ARNm dans un échantillon biologique provenant du patient, par hybridation de la séquence d'ARNm à SEQ ID NO : 1 ou SEQ ID NO : 2 dans des conditions de stringence élevée ; et
(b) la corrélation de la quantité de la séquence d'ARNm dans l'échantillon avec la présence d'un cancer de la prostate chez le patient.

11. Molécule d'acide nucléique isolée constituée d'un fragment de SEQ ID NO : 1, où le fragment comprend au moins 20 nucléotides contigus de SEQ ID NO : 1.

12. Molécule d'acide nucléique isolée selon la revendication 11, où l'acide nucléique comprend au moins 30 nucléotides contigus de SEQ ID NO : 1.

13. Molécule d'acide nucléique isolée selon la revendication 11, où l'acide nucléique comprend au moins 60 nucléotides contigus de SEQ ID NO : 1.

14. Procédé *in vitro* d'identification d'une lignée cellulaire réactive aux androgènes, le procédé comprenant :
(a) l'incubation d'une lignée cellulaire suspectée d'être réactive aux androgènes avec un androgène ; et
(b) la détection d'une séquence d'ARNm dans la lignée cellulaire, par hybridation de la séquence d'ARNm à SEQ ID NO : 1 ou SEQ ID NO : 2 dans des conditions de stringence élevée ;
dans lequel une augmentation de la séquence d'ARNm, comparativement à une lignée cellulaire non traitée, corrèle avec la lignée cellulaire étant réactive aux androgènes.

15. Procédé *in vitro* d'identification d'une lignée cellulaire réactive aux androgènes, le procédé comprenant :
(a) l'incubation d'une lignée cellulaire suspectée d'être réactive aux androgènes avec un androgène ;
(b) l'isolement d'une séquence d'ARNm à partir de ladite lignée cellulaire ;
(c) l'amplification d'un ADNc comprenant SEQ ID NO : 1 ou SEQ ID NO : 2 ou un fragment de celles-ci à partir de l'ARNm isolé en (b) ;
(d) l'incubation de l'ADNc amplifié avec une sonde d'acide nucléique qui s'hybride à SEQ ID NO : 1 ou SEQ ID NO : 2 pour former une molécule duplex qui est à la fois stable et sélective ; et
(e) la détection de l'hybridation entre l'ADNc amplifié et la sonde ;
dans lequel une augmentation de la séquence d'ARNm, comparativement à une lignée cellulaire non traitée, corrèle avec la lignée cellulaire étant réactive aux androgènes.

16. Procédé *in vitro* de mesure de la réactivité d'un tissu de la prostate à une hormonothérapie par ablation, le procédé comprenant :
(a) l'isolement d'ARNm à partir du tissu de la prostate après une hormonothérapie par ablation ;
(b) l'amplification d'un ADNc comprenant SEQ ID NO : 1 ou SEQ ID NO : 2 ou un fragment de celles-ci à partir de l'ARNm isolé en (a) ;
(c) l'incubation de l'ADNc amplifié avec une sonde d'acide nucléique qui s'hybride à SEQ ID NO : 1 pour former une molécule duplex qui est à la fois stable et sélective ; et
(d) la détection de l'hybridation entre l'ADNc amplifié et la sonde ;
dans lequel une diminution de la séquence d'ARNm, comparativement à du tissu de la prostate non traité, corrèle avec le tissu de la prostate étant réactif à une hormonothérapie par ablation.
